# EUROPEAN PATENT SPECIFICATION

(11) **EP 2 020 444 B1**
(45) Date of publication and mention of the grant of the patent: **31.05.2017**
(21) Application number: 07290980.7
(22) Date of filing: 03.08.2007
(51) Int. Cl.: C12N 15/867, A61K 39/00, A61K 39/12, C07K 14/16

(54) **Defective non-integrative lentiviral transfer vectors for vaccines**
Defekte nicht-integrative lentivirale Transfervektoren für Impfstoffe
Vecteurs lentivirus de transfert, déficient dans la function d'intégration, utilisés comme vaccins

(43) Date of publication of application: 04.02.2009
(73) Proprietor: INSTITUT PASTEUR, 75724 Paris Cedex 15 (FR)
(72) Inventor: Charneau, Pierre, 75005 Paris (FR); Coutant, Frédéric Philippe, 92130 Issy Les Moulineaux (FR)
(74) Representative: Desaix, Anne

(56) References cited:
- WO-A-2006/010834
- WO-A-2007/071994
- WO-A-2007/091066
- NEGRI DONATELLA R M ET AL: "Successful immunization with a single injection of non-integrating lentiviral vector." MOLECULAR THERAPY : THE JOURNAL OF THE AMERICAN SOCIETY OF GENE THERAPY SEP 2007, vol. 15, no. 9, 26 June 2007 (2007-06-26), pages 1716-1723, XP008087256 ISSN: 1525-0016
- PHILPOTT NICOLA J ET AL: "Use of nonintegrating lentiviral vectors for gene therapy" HUMAN GENE THERAPY, vol. 18, no. 6, June 2007 (2007-06), pages 483-489, XP002463956 ISSN: 1043-0342
- IGLESIAS MARIA CANDELA ET AL: "A single immunization with a minute dose of a lentiviral vector-based vaccine is highly effective at eliciting protective humoral immunity against West Nile virus" JOURNAL OF GENE MEDICINE, WILEY,, US, vol. 8, no. 3, March 2006 (2006-03), pages 265-274, XP002443147 ISSN: 1099-498X
- LEAVITT A D ET AL: "Human immunodeficiency virus type 1 integrase mutants retain in vitro integrase activity yet fail to integrate viral DNA efficiently during infection" JOURNAL OF VIROLOGY, THE AMERICAN SOCIETY FOR MICROBIOLOGY, US, vol. 70, no. 2, February 1996 (1996-02), pages 721-728, XP002207365 ISSN: 0022-538X
- Donatella Negri ET AL: "Successful immunization with a single injection on non-integrating lentiviral vector", Supplementary Material and methods Molecular Therapy, 26 June 2007 (2007-06-26), pages 1-6, XP055002820, Retrieved from the Internet: URL:http://www.nature.com/mt/journal/v15/n 9/suppinfo/6300241s1.html?url=/mt/journal/ v15/n9/full/6300241a.html [retrieved on 2011-07-14]
- FOLLENZI A ET AL: "Gene transfer by lentiviral vectors is limited by nuclear translocation and rescued by HIV-1 pol sequences", NATURE GENETICS, NATURE PUBLISHING GROUP, NEW YORK, US, vol. 25, no. 2, 1 June 2000 (2000-06-01), pages 217-222, XP002980776, ISSN: 1061-4036, DOI: 10.1038/76095
- BARRY S C ET AL: "Lentivirus vectors encoding both central polypurine tract and posttranscriptional regulatory element provide enhanced transduction and transgene expression", HUMAN GENE THERAPY, MARY ANN LIEBERT, NEW YORK ,NY, US, vol. 12, no. 9, 1 January 2001 (2001-01-01), pages 1103-1108, XP003004711, ISSN: 1043-0342, DOI: 10.1089/104303401750214311
- BUFFA VIVIANA ET AL: "Evaluation of a self-inactivating lentiviral vector expressing simian immunodeficiency virus gag for induction of specific immune responses in vitro and in vivo", VIRAL IMMUNOLOGY, MARY ANN LIEBERT, INC., NEW YORK, US, vol. 19, no. 4, 1 January 2006 (2006-01-01), pages 690-701, XP002482736, ISSN: 0882-8245, DOI: 10.1089/VIM.2006.19.690
- W. Wang ET AL: "A Role for Nuclear Factor I in the Intrinsic Control of Cerebellar Granule Neuron Gene Expression", Journal of Biological Chemistry, vol. 279, no. 51, 1 October 2004 (2004-10-01), pages 53491-53497, XP55053210, ISSN: 0021-9258, DOI: 10.1074/jbc.M410370200
- STRIPECKE R ET AL: "The use of lentiviral vectors in gene therapy of leukemia: Combinatorial gene delivery of immunomodulators into leukemia cells by state-of-the-art vectors", BLOOD CELLS, MOLECULES AND DISEASES, LAJOLLA, US, vol. 31, no. 1, 1 July 2003 (2003-07-01), pages 28-37, XP002532176, ISSN: 1079-9796, DOI: 10.1016/S1079-9796(03)00062-7
- SAUTER SYBILLE L ET AL: "Non-replicating viral vector-based AIDS vaccines: Interplay between viral vectors and the immune system", CURRENT HIV RESEARCH, BENTHAM SCIENCE PUBLISHERS, HILVERSUM, NL, vol. 3, no. 2, 1 April 2005 (2005-04-01), pages 157-181, XP009105561, ISSN: 1570-162X, DOI: 10.2174/1570162053506900
- Maciej Wiznerowicz: "TU vs pg of p24", , 14 August 2006 (2006-08-14), XP55172147, Retrieved from the Internet: URL:http://tronolab.epfl.ch/webdav/site/tr onolab/shared/protocols/TUvsp24.html [retrieved on 2015-02-25]
- FRÉDÉRIC COUTANT ET AL: "A Nonintegrative Lentiviral Vector-Based Vaccine Provides Long-Term Sterile Protection against Malaria", PLOS ONE, vol. 7, no. 11, 1 January 2012 (2012-01-01), pages e48644-e48644, XP055062725, ISSN: 1932-6203, DOI: 10.1371/journal.pone.0048644
- U S: "Guidance for Industry Estimating the Maximum Safe Starting Dose in Initial Clinical Trials for Therapeutics in Adult Healthy Volunteers Department of Health and Human Services Food and Drug Administration Center for Drug Evaluation and Research (CDER)", FOOD AND DRUG ADMINISTRATION, 1 July 2005 (2005-07-01), XP055152598,
- GERAERTS MARTINE ET AL: "Comparison of lentiviral vector titration methods", BMC BIOTECHNOLOGY, vol. 6, July 2006 (2006-07), ISSN: 1472-6750
- MAROZSAN ANDRE J ET AL: "Relationships between infectious titer, capsid protein levels, and reverse transcriptase activities of diverse human immunodeficiency virus type 1 isolates", JOURNAL OF VIROLOGY, vol. 78, no. 20, October 2004 (2004-10), pages 11130-11141, ISSN: 0022-538X
- SADOFF JERALD C ET AL: "Correlates, surrogates, and vaccines", JOURNAL OF INFECTIOUS DISEASES, vol. 196, no. 9, November 2007 (2007-11), pages 1279-1281, ISSN: 0022-1899
- Chapter 11: "The search for human RNA tumor viruses" In: "RNA Tumor Viruses: Molecular Biology of Tumor Viruses", 1982, Cold Spring Harbor Laboratory Press pages 1205-1281,

## Description

### BACKGROUND

Prevention of infectious diseases through vaccine development is one of the greatest achievements of modern medicine. Nonetheless, considerable challenges remain for the development of new vaccines combining efficiency with enhanced safety profiles. Recently, lentiviral vectors (LV), such as human immunodeficiency virus (HIV)-1-derived lentiviral vectors, have emerged as promising vaccination tools. These vectors elicit both specific cytotoxic and strong humoral immune responses in several animal models. These properties, and especially the efficiency of the adaptive immunity, rely in part on the fact that integrative LV can mediate efficient gene transfer and integration into dendritic cells (DC), allowing a sustained presentation of tumoral or viral antigens (Ags) and subsequent activation of naive T cells. Moreover, problems of vector-specific immunity are largely reduced with the use of LV because of the absence of pre-existing immunity in humans and by the fact that LV do not contain genes encoding HIV-1 viral proteins, which allows repeated immunizations.

Although integrative LV-based vaccines have proven to elicit specific and protective immunity against tumor and infectious agents, their transition as vaccinal vectors from preclinical evaluation to clinical development is hindered by the problem of potential insertional mutagenesis linked to the pseudo-randomly integration of the transgene in the genome of the transduced cells. These integration events can cause severe side-effects, as unfortunately illustrated by the three leukaemia cases in the SCID-X1 gene therapy trial, in which transformed cell-growth was triggered by the integration of the Moloney-derived retroviral vector at close proximity of the LMO2 proto-oncogene. It has been further demonstrated that oncogenesis was the consequence of an enhancer proximity from the vector sequence on the LMO2 proto-oncogene.

Several arguments point out that the use of integrative LV, especially in the field of vaccination, holds lower risks of potential oncogenesis linked to insertional mutagenesis events. Firstly, in a vaccination scenario based on direct injection of Ag-encoding integrative LV, transduced cells that express the relevant Ag become targets of the elicited immune response and are thought to be eliminated rapidly from the vaccinated organism. Secondly, the deletion in the 3' LTR of the viral promoter and enhancer sequences in self-inactivating LV limits the likelihood of endogenous promoter activation. Thirdly, although many lymphocytes of HIV-infected patients carry a large number of transcriptionally active HIV integrated proviruses, it is not recognized that the integration events that occur in infected cells cause oncogenesis. Consistently, a recent study has provided evidences that LV transduction, even at high integration loads do not accelerate tumorigenesis in a tumor-prone murine model. Nevertheless, investigations to resolve the problem of insertional mutagenesis need to be actively pursued since safety considerations are the major limitations for the agreement of LV in clinical development. In this context, the development of LV that allows a targeted integration or does not integrate at all would provide an important step toward the development of fully safe vectors.

Patent application WO2006/010834 reports the design and use of integration-deficient lentivirus (LV). These vectors carry a defective integrase with the mutation D64V in the catalytic domain of the enzyme, which specifically blocks the DNA cleaving and joining reactions of the integration step. The D64V mutation decreases integration of pseudotyped HIV-1 up to 1/10,000 of wild type, but keep their ability to transduce nondividing cells, allowing efficient transgene expression. Human immunodeficiency virus type 1 integrase mutants retain *in vitro* integrase activity yet fail to integrate viral DNA efficiently during infection (Leavitt et al. J. Virol. 1996 Feb; 70(2): 721-8). These vectors have been demonstrated to efficiently transduce primary cells such as neuronal or retinal cells and to rescue representative models of retinal degeneration. However, in this patent application, studies have been limited to targeting of retina and neuronal cells, and expression of a transgene in these cells. The efficiency of such vectors, in complex biological mechanisms involving cell-cell interaction, such as immune response, has been shown in Negri et al. 2007, Molecular Therapy, 15(9), p.1716. However, the dose required to obtain an immune response which protects against challenge with a pathogen has not been disclosed.

There is thus a need for methods to initiate specific and effective immune responses, with secure and specific vectors. The present invention fulfils this need by providing non-integrating vectors (also designated as non-integrative-vectors or integration-incompetent vectors) comprising a defective integrase protein and further comprising a RNA genome which is the transcription product of a vector genome comprising a polynucleotide encoding at least one antigenic polypeptide, two LTRs and a DNA Flap comprising a cPPT and a CTS domains, in the manufacture of an immunogenic composition for eliciting a protective immune response against said at least one antigenic polypeptide to prevent infection by a pathogen or malignant states, in a human patient administered with said immunogenic composition comprising doses of said HIV lentiviral vector ranging from 1 µg to 100 µg of p24 antigen, wherein said defective integrase protein is an integrase having a class I mutation to specifically alter its integrase activity. The description also discloses non-integrating vectors as vaccination tools, and their use for inducing or eliciting an immune response, and especially a protective humoral immune response. Indeed, in view of the non-integrative behaviour of the used vectors, the question was asked as to whether the encoded antigenic polypeptide would be produced in sufficient quantity and/or over sufficient time to enable an efficient presentation to immune cells, and finally an antigen-specific and effective immune response. It was thus assayed whether the induction of immune responses, such as antigen-specific immune responses, in vaccine strategies involving lentiviral vectors should occur despite failure of said vector to integrate, since main target cells are non-dividing DC. The present invention shows that non integrative lentiviral vectors (LV) are not only efficient in transducing immune cells, such as DC, but also that a single immunization with these vectors expressing an antigenic polypeptide, for example the secreted form of the West Nile Virus envelope, can prime immune response and confers protection against lethal challenge.

### BRIEF DESCRIPTION OF THE FIGURES

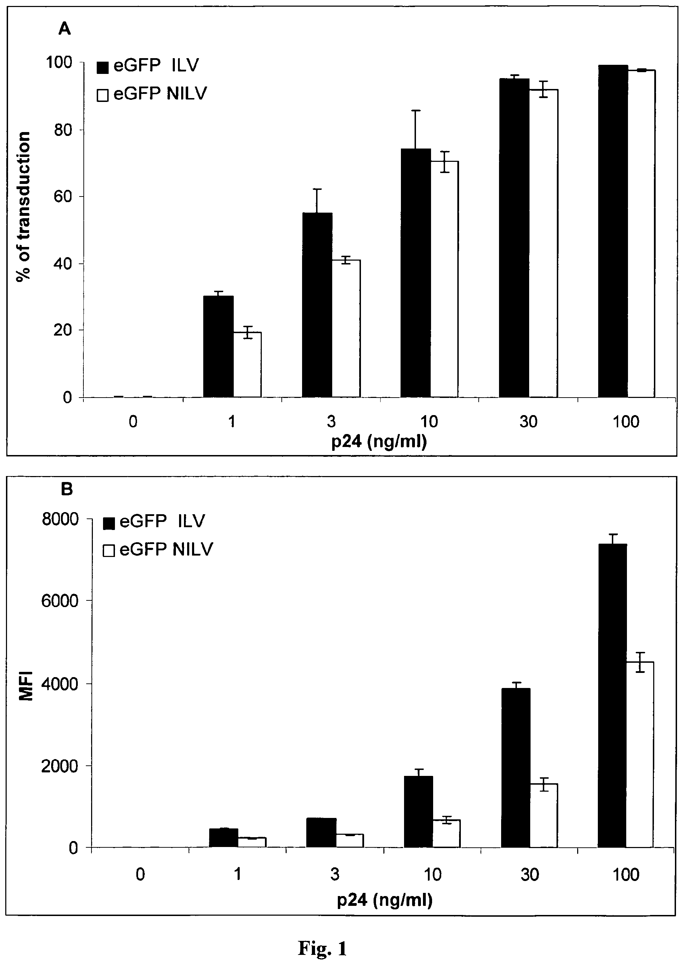

**Figure 1****: Efficient transduction of nondividing cells with LV defective for integration.** Aphidicolin-treated HeLa cells were transduced with graded doses (from 1 to 100 ng of p24 antigen per ml of medium) of eGFP-integrative LV (eGFP-ILV) or eGFP-nonintegrative LV (eGFP-NILV). At 48 hours post-transduction, eGFP expression was analyzed by flow cytometry. The upper panel shows the percentage of GFP positive cells and the lower panel shows MFI (mean fluroescent intensity) of the GFP positive cells.

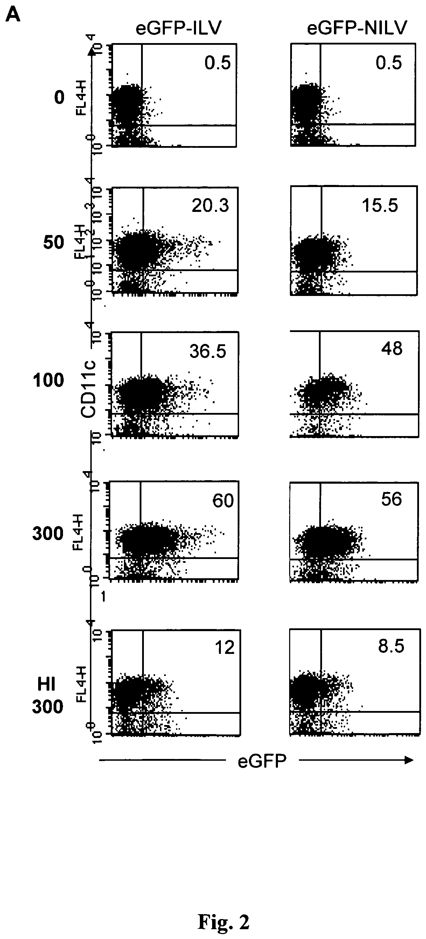

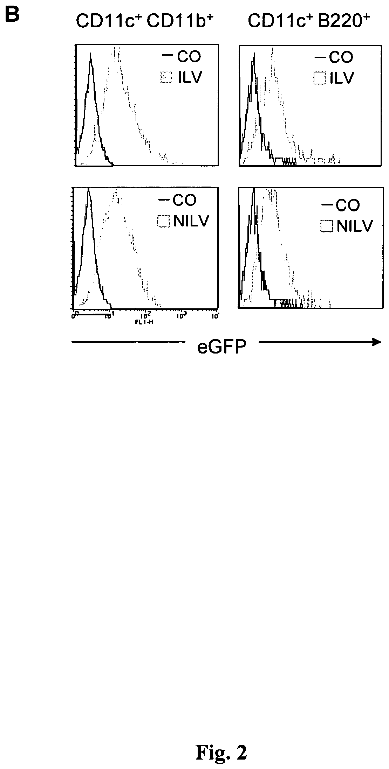

**Figure 2****: Lentiviral vector transduction leads to effective antigen expression both in conventional dendritic cells (cDC) and in plasmacytoid DC (pDC).** (A) Dose-response transduction experiments (from 0 to 300 ng/ml) with eGFP-integrative LV (eGFP-ILV) or eGFP-non integrative LV (eGFP-NILV) or with 300 ng/ml of heat-inactivated (HI) eGFP-ILV or eGFP-NILV. On day 6, FL-DC were exposed to vector particles for 48 hours and transduction of CD11c positive cells was assessed by measuring eGFP expression by flow cytometry. Numbers indicate the percentage of CD11c cells expressing eGFP. (B) Transduction of pDC and cDC by LV. Expression of eGFP by cDC (CD11c⁺ B220⁻) and pDC (CD11c⁺ B220⁺) is shown. Thin lines, control cells; filled profiles, FL-DC transduced with 300 ng/ml of vector particles.

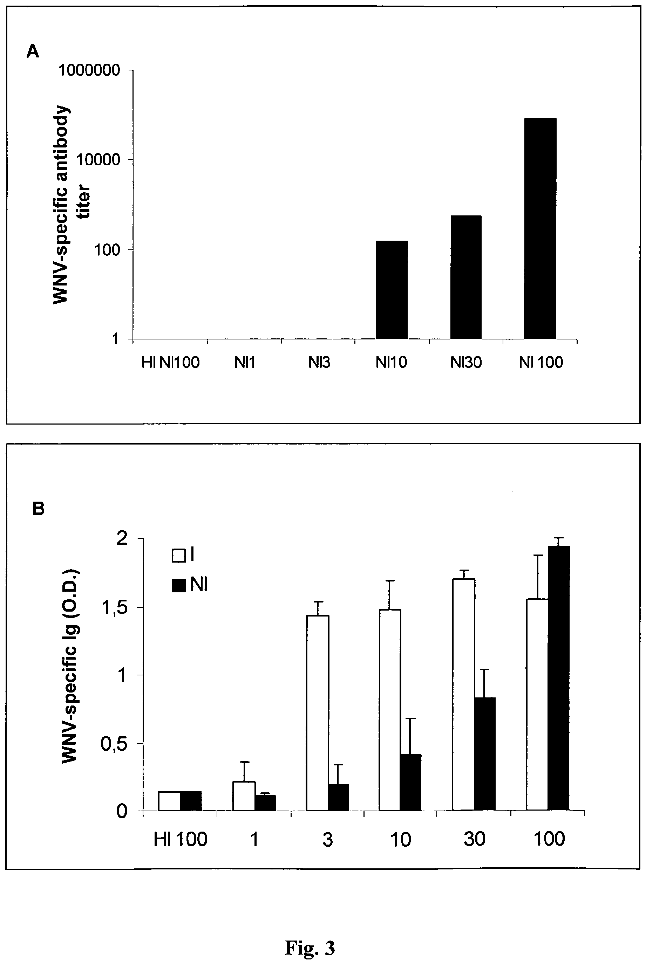

**Figure 3****: A single dose of ₛE_{WNV}-NILV elicits a strong and specific antibody response.** Groups of adult mice were immunized i.p. with graded doses of ₛE_{WNV}-NILV (from 1 to 100 ng of p24 antigen) (A, B) or ₛE_{WNV}-ILV (B). Control mice were injected with heat-inactivated ₛE_{WNV}-LV NI (A, B) or I (B) (HI 100). After 21 days, pooled sera (6 mice per group) were assessed for WNV-specific antibodies.

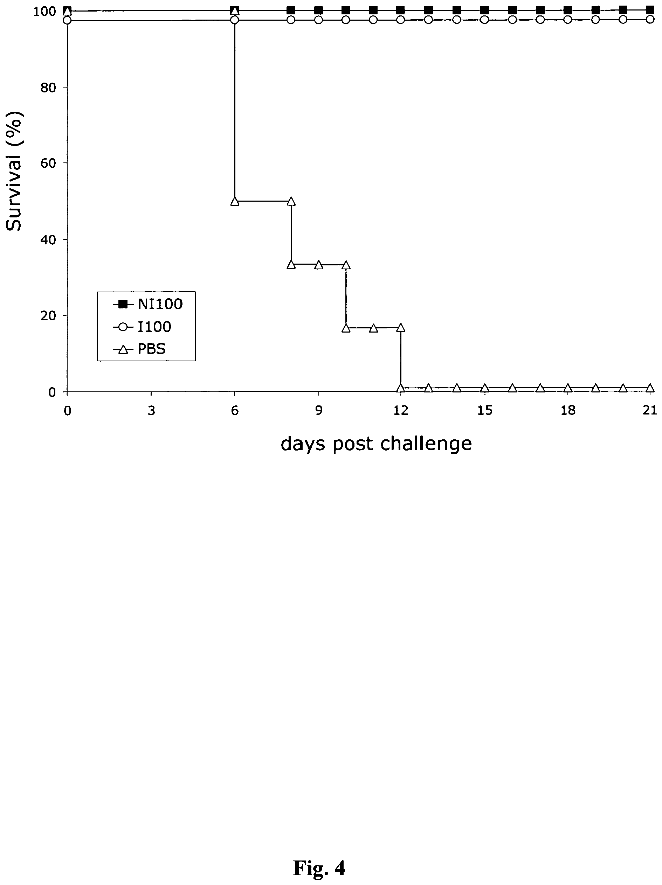

**Figure 4****: Rapid protection against WNV infection conferred by sEwnv-NILV immunization.** Six mice/group were vaccinated with 100ng of sEwnv-NILV or 100ng of sEwnv-ILV. A control group of mice inoculated with phosphate-buffered saline (PBS) was included. One week after the vaccination, mice were challenged with 1,000 i.p. LD₅₀s of WNV strain IS-98-ST1. Survival was recorded for 21 days.

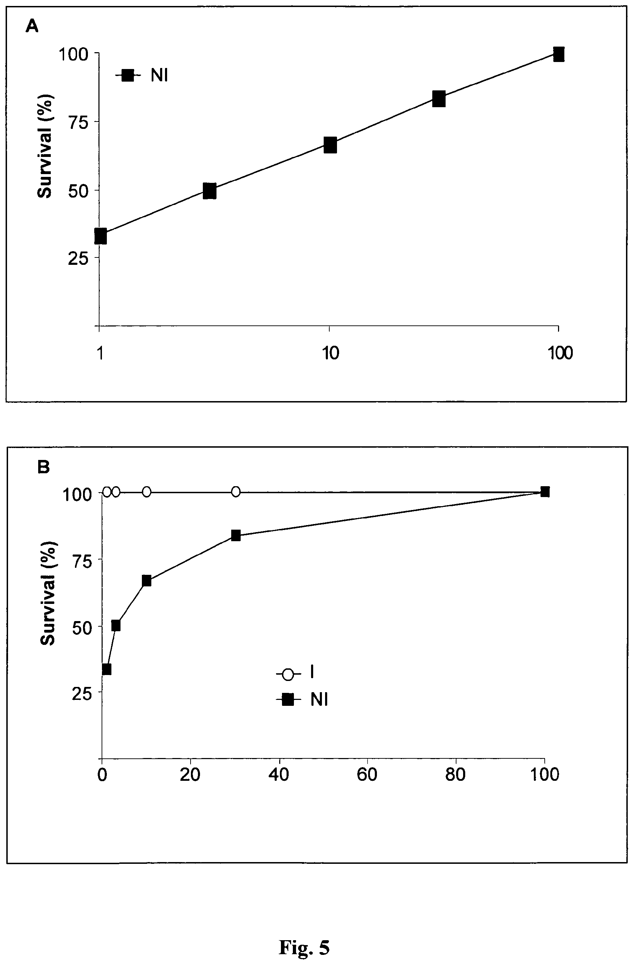

**Figure 5****: Efficient long-term protection by sE_{WNV}**-**NILV against WNV infection.** Two months post-immunization with graded doses of sE_{WNV}-NILV (1-100 ng of p24 antigen) (A, B) or ₛE_{WNV}-ILV (B), mice were inoculated with 1,000 i.p. LD₅₀s of WNV strain IS-98-ST1. Survival was recorded for 21 days.

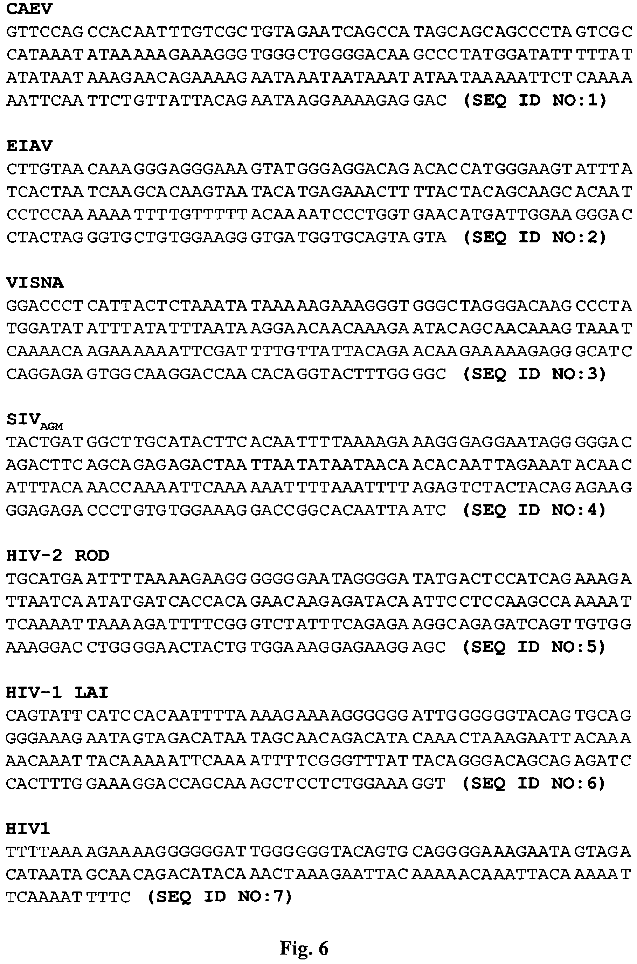

**Figure 6****: Nucleotide sequences of various retrovirus DNA FLAP** as defined in different viruses: CAEV (SEQ ID NO:1), EIAV (SEQ ID NO:2), VISNA (SEQ ID NO:3), SIV AGM (SEQ ID NO:4), HIV-2 RID (SEQ ID NO:5), HIV-1 LAI (SEQ ID NO:6) and HIV-1 (SEQ ID NO:7).

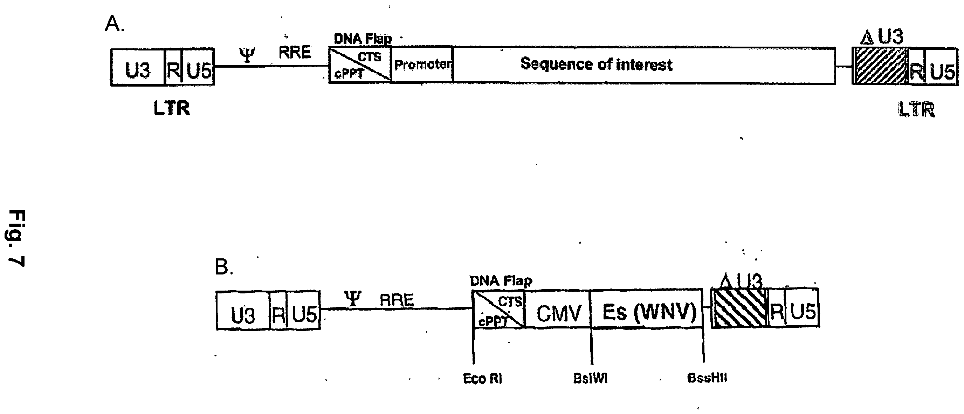

**Figure 7****: (A) vector genome construct organization for the purpose of the invention, based on a typical HIV-1 genome sequence; (B) Schematic representation of the TRIP/sEwnv vector.** The following abbreviations are used: U3, R and U5 represent the domains of the LTR; ΔU3: deletion of the U3 domain: RRE: Rev-responsive element; ψ: encapsidation signal; cPPT and CTS represent the DNA flap; CMVie: cytomegalovirus immediate early promoter.

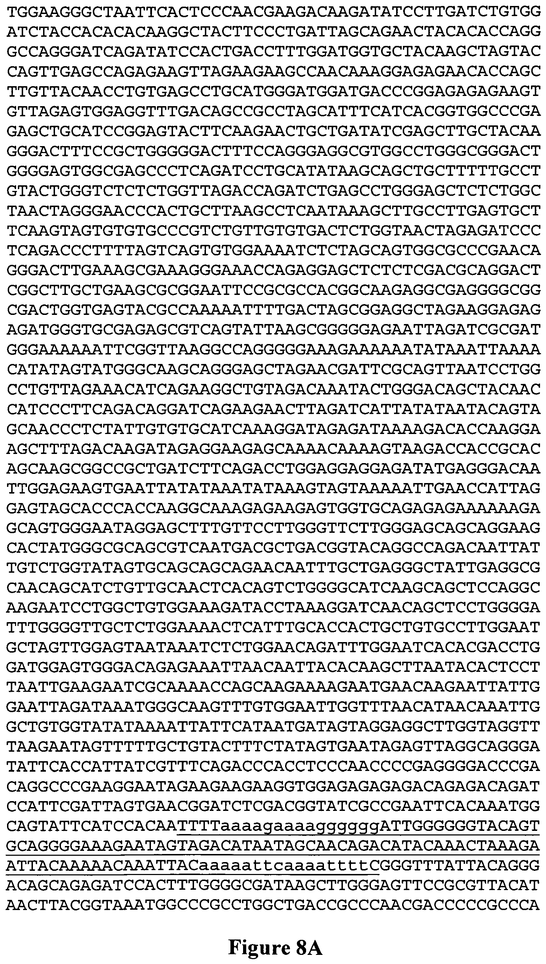

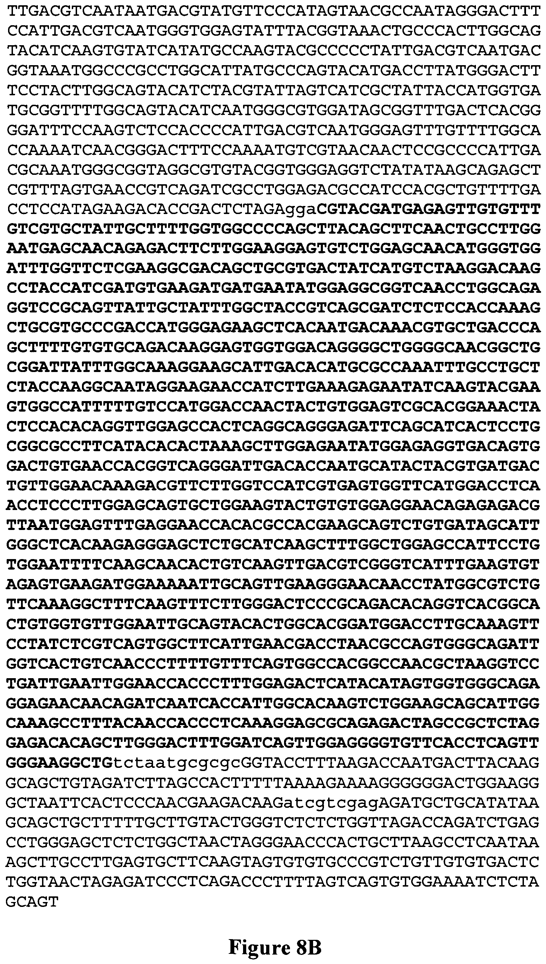

**Figure 8****: Nucleotide sequence of the TRIPsEwnv vector.** The cPPT/CTS region is underlined. In this region, cPPT and CTS domains appear in lowercase. The sEwnv sequence, represented in bold, is a BsiWI-BssHII DNA insert. This vector has been deposited at the CNCM (Paris, France), under number I-3076, on August 27, 2003.

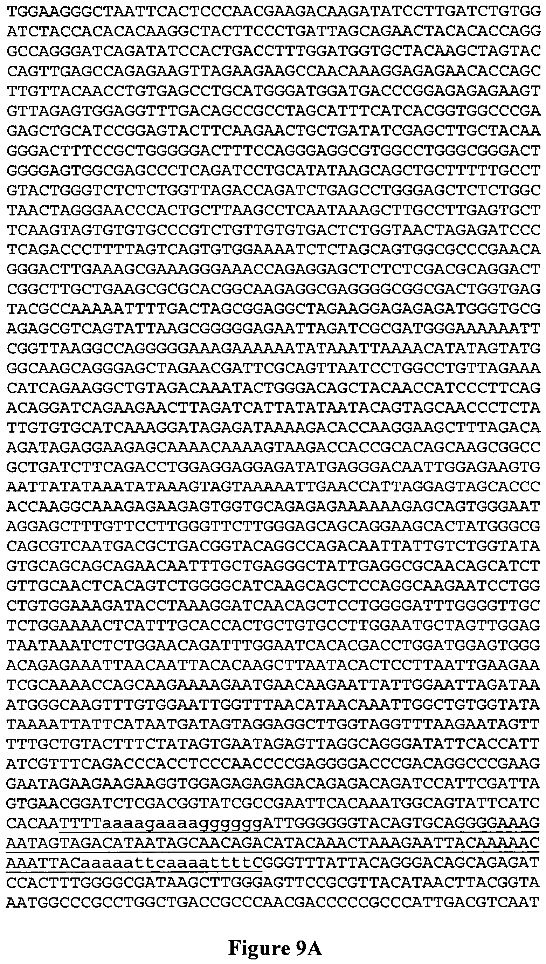

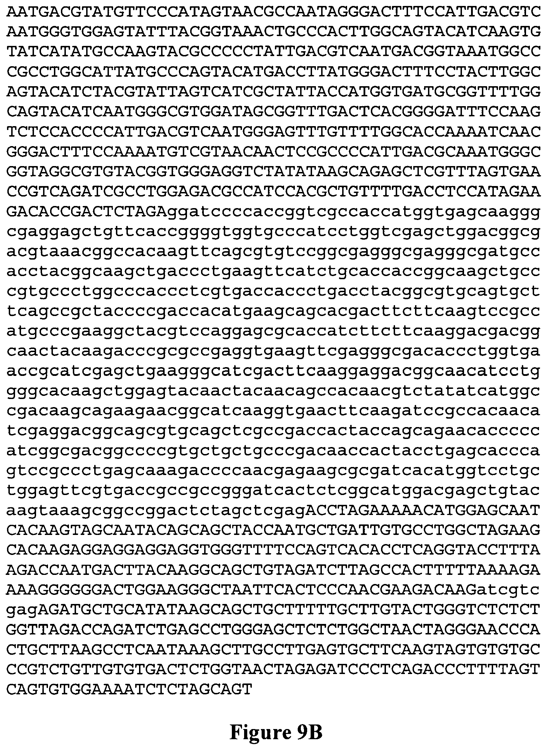

**Figure 9****: Nucleotide sequence of the TRIP GFP vector.** The cPPT/CTS region is underlined. In this region, cPPT and CTS domains appear in lowercase. The GFP sequence is located between nucleotides 2816 and 3573. This vector has been deposited at the CNCM, under number I-2330, on October 11, 1999 (pTRIP [deltaU3] CMV GFP).

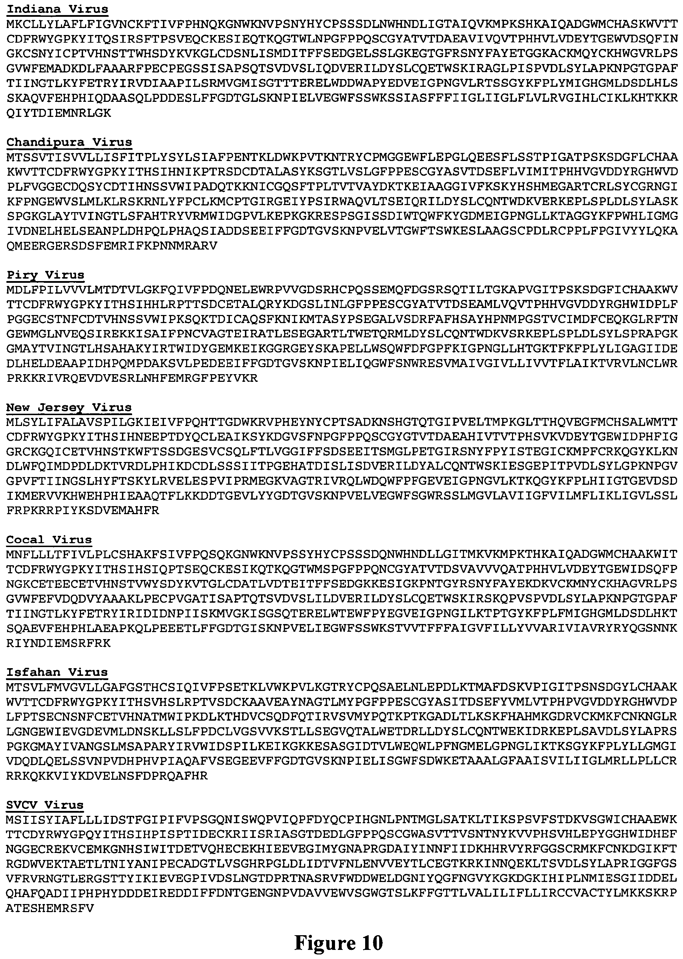

**Figure 10****: VSV-G protein sequences from various serotypes known in the Vesiculovirus genus for VSV species:** Indiana (NCBI Accession Number J02428), Chandipura (J04350), Piry (D26175), New Jersey, Cocal (AF045556), Isfahan (AJ810084) and Spring viremia of carp virus (SVCV)(AY527273).

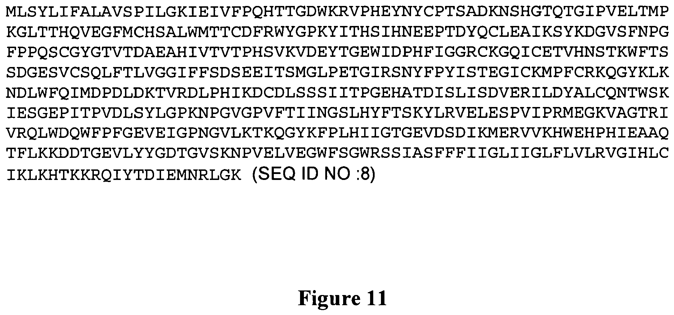

**Figure 11****: Recombinant envelope of the VSV obtained by recombining sequences of the New Jersey and Indiana serotypes.** The VSV-G ECTO (NJ)-TM (IND) contains the transmembrane domain of the Indiana VSV-G and the ectodomain of the New-Jersey serotype (SEQ ID NO:8).

### DETAILED DESCRIPTION

The present invention relates to the use of a lentiviral vector wherein the expressed integrase protein is defective and which further comprises a polynucleotide encoding at least one antigenic polypeptide, to produce an immunogenic composition suitable for eliciting an immune response against said at least one polypeptide, in a host in need thereof. Such use of such a lentiviral vector are encompassed within the invention inasmuch as they are comprised in the scope of the claims.

The expression "*lentiviral vecto*r*"* encompasses a lentiviral vector particle that comprises both proteins and genetic material, preferably encapsidated into these proteins. Particles are made of viral envelope proteins (encoded by an *env* gene) as well as structural proteins (encoded by a *gag* gene). Inside the particles, a viral core (or capsid) formed of three enzymes (encoded by a *pol* gene), *i.e.*, the reverse transcriptase, the integrase and the protease, and genetic material (lentiviral genome). In the present invention, it is essential that the integrase protein is a defective integrase protein.

By "*defective*"*,* it is meant that the integrase, preferably of lentiviral origin, is devoid of the capacity of integration of the lentiviral genome into the genome of the host cells *i.e.,* an integrase protein mutated to specifically alter its integrase activity. Accordingly the integrase capacity of the protein is altered whereas the correct expression of the GAG, PRO and POL proteins and/or the formation of the capsid and hence of the vector particles, as well as other steps of the viral cycle, preceding or subsequent to the integration step, such as the reverse transcription, the nucleus import, stay intact. An integrase is said defective when the integration that it should enable is altered in such a way that an integration step takes place less than 1 over 1000, preferably less than 1 over 10000, when compared to a lentiviral vector containing a corresponding wild-type integrase.

The property of the integrase of being defective, results from a mutation of class 1, preferably amino acid substitutions (one-amino acid substitution) or short deletions giving rise to a protein fulfilling the requirements of the preceding paragraph. The mutation is carried out within the pol gene. Examples of mutations altering HIV-1 and enabling to obtain a non-functional integrase for integration (integration-incompetent integrase) are the following: H12N, H12C, H16C, H16V, S81R, D41A, K42A, H51A, Q53C, D55V, D64E, D64V, E69A, K71A, E85A, E87A, D116N, D116I, D116A, N120G, N120I, N120E, E152G, E152A, D35E, K156E, K156A, E157A, K159E, K159A, K160A, R166A, D167A, E170A, H171A, K173A, K186Q, K186T, K188T, E198A, R199C, R199T, R199A, D202A, K211A, Q214L, Q216L, Q221L, W235F, W235E, K236S, K236A, K246A, G247W, D253A, R262A, R263A and K264H. Another proposed substitution is the replacement of the amino acids residues RRK (positions 262 to 264) by the amino acids residues AAH. In a particular embodiment, the following substitutions are preferred: H12N, H12C, H16C, H16V, S81R, D41A, K42A, H51A, Q53C, D55V, D64E, D64V, E69A, K71A, E85A, E87A, D116I, D116A, N120G, N120I, N120E, E152G, E152A, D35E, K156E, K156A, E157A, K159E, K159A, K160A, R166A, D167A, E170A, H171A, K173A, K186Q, K186T, K188T, E198A, R199C, R199T, R199A, D202A, K211A, Q214L, Q216L, Q221 L, W235F, W235E, K236S, K236A, K246A, G247W, D253A, R262A, R263A and K264H. A particularly proper mutation is the D64V mutation that has been shown, within the present invention, to provide good results in vaccination studies.

The envelope protein of the lentiviral vector for use according to the invention may be pseudotyped with the envelope protein of the lentivirus used to prepare the lentiviral vector, or alternatively with a heterogeneous envelope protein that is chosen with respect to the cells to be targeted into the host.

In a preferred embodiment, said lentiviral vector is pseudotyped with a VSV-G protein. The VSV-G glycoprotein may originate from different serotypes of the genus of the vesiculoviruses: VSV-Indiana serotype, VSV-New Jersey serotype or other glycoproteins of the vesiculoviruses such as Piry, Chandipura, Isfahan and Cocal (Figure 10). The VSV-G glycoprotein is chosen among species classified in the vesiculovirus genus: Carajas virus (CJSV), Chandipura virus (CHPV), Cocal virus (COCV), Isfahan virus (ISFV), Maraba virus (MARAV), Piry virus (PIRYV), Vesicular stomatitis Alagoas virus (VSAV), Vesicular stomatitis Indiana virus (VSIV) and Vesicular stomatitis New Jersey virus (VSNJV) and/or Grass carp rhabdovirus, BeAn 157575 virus (BeAn 157575), Boteke virus (BTKV), Calchaqui virus (CQIV), Eel virus American (EVA), Gray Lodge virus (GLOV), Jurona virus (JURV), Klamath virus (KLAV), Kwatta virus (KWAV), La Joya virus (LJV), Malpais Spring virus (MSPV), Mount Elgon bat virus (MEBV), Perinet virus (PERV), Pike fry rhabdovirus (PFRV), Porton virus (PORV), Radi virus (RADIV), Spring viremia of carp virus (SVCV), Tupaia virus (TUPV), Ulcerative disease rhabdovirus (UDRV) and Yug Bogdanovac virus (YBV). In a particular embodiment, the VSV-G protein originating from a VSV is modified with respect to its native form, especially to improve pseudotyping.

In a particular embodiment, the envelope protein comprises domains or fragments originating from different envelope protein(s) of different viruses, especially of different genus of different species of VSV. In the case of VSV, the G protein comprises or consists of the transmembrane domain of the indiana VSV and the ectodomain of a strain of a different VSV serotype. In a particular embodiment, the envelope protein(s) comprises the transmembrane domain of the indiana VSV and the ectodomain of the New-Jersey VSV (Figure 11; SEQ ID NO:8).

Appropriate other modifications encompass mutations, especially point mutations that improve pseudotyping. Such mutations for the VSV-G proteins may be carried out in the transmembrane domain by substituting or deleting one or several amino acid residues. Other examples of mutations are disclosed in Fredericksen B.L. et al (J. Virol. (1995) 69: 1435-1443) or Nishimura N. et al (PNAS (2002) 99: 6755-6760).

In another aspect, said lentiviral vector is pseudotyped with HA protein (influenza-hemaglutinin), RD114 protein, modified envelopes with inserted cell-specific ligands or with viral envelope proteins originated from a virus selected in one or several of the following orders or families: Arenaviridae, Flaviridae, Togaviridae, Coronaviridae, Orthomyxoviridae, Retroviridae and Mononegavirales including Paramyxoviridae, Rhabdoviridae or Filoviridae. Any virus envelope protein is suitable for pseudotyping, assuming that the pseudotyping is compatible with production and purification steps of lentiviral vector particles.

Particular cells targeted by the lentiviral vectors for use according to the present invention are cells involved in immune response, such as antigen presenting cells (APC), dendritic cells (DC), including conventional DC (cDC) or plasmacytoid (pDC), T cells, including CD4⁺ or CD8⁺, B cells, monocytes, macrophages, Natural Killer cells, Natural Killer T cells, endothelial cells and epithelial cells. Interestingly, B cells have been recently shown to interact with circulating mature DC, thus activating these B cells, that in turn efficiently present antigens to naïve T cells (amplification of the mature APC population); therefore, this points out the critical role of B cells in priming cells involved in cellular immune response, and particularly naïve CD8+ T cells (Diaz de Durana; 2006).

The polynucleotide encoding at least one antigenic polypeptide is encompassed within the lentiviral vector genome *i.e.,* the genetic material of the lentiviral vector (i.e., in the particle). The expression "vector genome" refers to the nucleic acid which is suitable for constituting the genome of the lentiviral vector particles. Accordingly the expression relates to any appropriate nucleic acid, *i.e.,* DNA or RNA, either double or single stranded, including in the form containing the DNA flap as a triplex sequence, depending upon the stage of cycle of the particles, including the vector plasmid - used for cotransfection of the host cells with the encapsidation plasmid and the envelope plasmid - for expression of the particles, or the RNA genome of the particles when formed, or the various forms the of nucleic acid of this genome in the transduced cells of the host to whom particles are administered, including the vector pre-integration complex.

The structure and composition of the vector genome used to prepare the lentiviral vectors for use according to the invention are in accordance with those described in the art. Especially, minimum lentiviral gene delivery vectors can be prepared from a vector genome, which only contains, apart from the heterologous polynucleotide of therapeutic interest, the sequences of the lentiviral genome which are non-coding regions of said genome, necessary to provide recognition signals for DNA or RNA synthesis and processing. Hence, a vector genome may be a replacement vector in which all the viral coding sequences between the 2 long terminal repeats (LTRs) have been replaced by the polynucleotide of interest. However, said vector genome also comprises in addition, a polynucleotide consisting in the DNA flap

The DNA flap (defined in Zennou V. et al 2000, Cell vol 101, 173-185 or in WO 99/55892 and WO 01/27304), is a structure which is central in the genome of some lentiviruses especially in HIV retroviruses, where it gives rise to a 3-stranded DNA structure normally synthesized during especially HIV reverse transcription and which acts as a cis-determinant of HIV genome nuclear import. The DNA flap enables a central strand displacement event controlled in cis by the central polypurine tract (cPPT) and the central termination sequence (CTS) during reverse transcription. When inserted in lentiviral derived vectors, the polynucleotide enabling the DNA flap to be produced during retro-transcription, stimulates gene transfer efficiency and complements the level of nuclear import to wild-type levels (Arhel N. et al, Retrovirology 2006, 3:38, 26 June 2006, *Wild-type and central DNA flap defective HIV-1 lentiviral vector genomes: intracellular visualization at ultra structural resolution levels).*

In a particular aspect, the DNA flap is inserted immediately upstream of the polynucleotide encoding at least one antigenic polypeptide, advantageously to have a central position in the vector genome. A DNA flap suitable for use in the invention may be obtained from a retrovirus, especially from a lentivirus, or from a retrovirus-like organism such as retrotransposon, either prepared synthetically (chemical synthesis) or by amplification of the DNA flap from any retrovirus especially from a lentivirus nucleic acid such as by Polymerase chain reaction (PCR). The DNA flap may be obtained from a retrovirus, especially a lentivirus, especially from a human retrovirus or lentivirus and in particular a HIV retrovirus, or from the CAEV (Caprine Arthritis Encephalitis Virus) virus, the EIAV (Equine Infectious Anaemia Virus) virus, the VISNA virus, the SIV (Simian Immunodeficiency Virus) virus or the FIV (Feline Immunodeficiency Virus) virus. In a more preferred embodiment, the DNA flap is obtained from an HIV retrovirus, for example HIV-1 or HIV-2 virus including any isolate of these two types. Preferred DNA flap comprises or consists in the sequences as defined in SEQ ID NO: 1 to 7 (Figure 6). It is noteworthy that the DNA flap is used as a DNA fragment isolated from its natural (viral genome) nucleotide context *i.e.,* out of the context of the *pol* gene in which it is naturally contained in the lentivirus genome. Therefore, the DNA flap is used, in the present invention, deleted from the unnecessary 5' and 3' parts of the *pol* gene and is recombined with sequences of different origin. The DNA flap may be either prepared synthetically (chemical synthesis) or by amplification of the DNA providing the DNA flap from the appropriate source as defined above such as by Polymerase chain reaction (PCR). In a more preferred embodiment, the DNA flap is obtained from an HIV retrovirus, for example HIV-1 or HIV-2 virus including any isolate of these two types.

They are preferably inserted in the vector genome in a position which is central or nearly central to said vector genome. Alternatively they may be inserted immediately upstream from the promoter controlling the expression of the polynucleotide.

According to a particular aspect, a DNA flap has a nucleotide sequence of about 90 to about 140 nucleotides. In HIV-1, the DNA flap is a stable 99-nucleotide-long plus strand overlap. When used in the genome vector of the lentiviral vector for use according to the invention, it may be inserted as a longer sequence, especially when it is prepared as a PCR fragment. A particular appropriate polynucleotide comprising the structure providing the DNA flap is a 178-base pair polymerase chain reaction (PCR) fragment encompassing the cPPT and CTS regions of the HIV-1 DNA

The lentiviral vector genome may comprise regulatory signals for transcription and expression of non lentiviral origin, such as a promoter and/or an enhancer. Examples of promoters that can be used in immune response elicitation are CMV also referred to as CMVie (CMV immediate early), EF1α promoter, CGA promoter, CD11c promoter and house keeping gene promoters such as PGK promoter, ubiquitin promoter, actin promoter, histone promoter, alpha-tubulin promoter, beta-tubulin promoter, superoxide dismutase 1 (SOD-1) promoter, dihydrofolate reductase (DHFR) promoter, hypoxanthine phosphorybosyltransferase (HPRT) promoter, adenosine deaminase promoter, thymidylate synthetase promoter, dihydrofolate reductase P1 promoter, glucose-6-phosphate sehydrogenase promoter or nucleolin promoter. In a particular aspect, said polynucleotide encoding said at least one antigenic is under the control of regulatory signals for transcription and expression.

Said lentiviral vector genome comprises all the elements necessary for the nucleic import and the correct expression of the polynucleotide encoding at least one antigenic polypeptide. As examples of elements that can be inserted in the lentiviral genome of the lentiviral vector for use according to the invention are at least two long terminal repeats (LTR), such as a LTR5' and a LTR3', a psi sequence involved in the lentiviral genome encapsidation, and at least one DNA flap comprising a cPPT and a CTS domains.

In a particular aspect, the LTR, preferably the LTR3', is deleted for the promoter and the enhancer of U3; this modification has been shown to increase substantially the transcription of the transgene inserted in the lentiviral genome (WO01/27304).

The lentiviral vector genome may also comprise elements selected among a splice donor site (SD), a splice acceptor site (SA) and/or a Rev-responsive element (RRE). In a particular aspect, the vector genome may further comprise one or several unique restriction sites for cloning the polynucleotide encoding said at least one antigenic polypeptide.

In a particular embodiment of the invention, the lentiviral vector genome further comprises an origin or replication (ori), whose sequence is dependent on the nature of cells where the lentiviral genome has to be expressed. Said origin of replication may be from eukaryotic origin, preferably of mammalian origin, most preferably of human origin. It is an advantageous embodiment of the invention to have an origin or replication inserted in the lentiviral genome of the lentiviral vector of the invention. Indeed, since the lentiviral genome does not integrate into the cell host genome (because of the defective integrase), the lentiviral vector genome is lost in cells undergoing frequent cell divisions; this is particularly the case in immune cells, such as B or T cells. The presence of an origin of replication ensures that at least one lentiviral genome is present in each cell, even after cell division, maximazing the efficiency of the immune response.

In addition to the elements quoted above, the lentiviral genome may also comprise at least one scaffold attachment region (SAR) and/or a matrix attachment region (MAR). Indeed, these AT-rich sequences enable to anchor the lentiviral genome to the matrix of the cell chromosome, thus regulating the transcription of the polynucleotide encoding at least one antigenic polypeptide, and particularly stimulating gene expression of the transgene and improving chromatin accessibility.

In particular embodiments of the invention, either independently of or in combination with the embodiments discussed throughout the specification, the lentiviral vector genome is devoid of functional *gag, pol* and/or *env* lentiviral genes. By "*functional*" it is meant a gene that is correctly transcribed, and/or correctly expressed. Thus, the lentiviral vector genome of the invention in this embodiment contains at least one of the gag, pol and env genes that is either not transcribed or incompletely transcribed; the expression "*incompletely transcribed*" refers to the alteration in the transcripts gag, gag-pro or gag-pro-pol, one of these or several of these being not transcribed. In a particular embodiment, the lentiviral genome is devoid of *gag, pol* and/or *env* lentiviral genes.

In a particular embodiment the lentiviral vector genome is also devoid of the coding sequences for Vif-, Vpr-, Vpu- and Nef-accessory genes (for HIV-1 lentiviral vectors), or of their complete or functional genes.

The lentiviral vector for use according to the invention is non replicative *i.e*., the vector and lentiviral vector genome are not able to form new particles budding from the infected host cell. This may be achieved by the absence in the lentiviral genome of the *gag, pol* or *env* genes, as indicated in the above paragraph; this can also be achieved by deleting other viral coding sequence(s) and/or cis-acting genetic elements needed for particles formation. The absence of replication of the lentiviral vector should be distinguished from the replication of the lentiviral genome. Indeed, as described before, the lentiviral genome may contain an origin of replication ensuring the replication of the lentiviral vector genome without ensuring necessarily the replication of the vector (or particle).

In a further aspect, particularly when the polynucleotide encoding the at least one antigenic polypeptide originates from a lentivirus, said lentiviral vector genome does not comprise a complete lentiviral *gag, pol* or *env* coding polynucleotide, meaning that said lentiviral vector genome comprises a polynucleotide shorter than the lentiviral *gag, pol* or *env* genes. Therefore, the *gag* coding sequence is shorter than 1500 pb for HIV-1 or HIV-2 ; the *pol* coding sequence is shorter than 3000 pb for HIV-1 and 3300 pb for HIV-2 ; the *env* coding sequence is shorter than 2700 pb for HIV-1 and 2500 pb for HIV-2. This size refers to the longest continuous nucleotide sequence found as such in the native lentiviral genome. However, in another particular embodiment, the lentiviral genome is devoid of all endogenous coding lentiviral sequences.

The polynucleotide contained in the lentiviral vector genome encodes at least one antigenic polypeptide. A polypeptide is said antigenic when its sequence contains at least one peptide (epitope) able to elicit an immune response when put in contact with antigen presenting cells (APC). The epitope may depend either from a specific three-dimensional antigenic conformation (conformational epitope), or may correspond to a simple primary sequence region (linear epitope). The size of the polypeptide ranges from at least 9 amino acids up to 500 amino acids, and is preferably less than 200 amino acids.

Preferred antigenic polypeptides encompass at least one B epitope, capable of eliciting a humoral immune response, particularly a protective humoral response, or a T-epitope capable of eliciting a cellular immune response.

In a particular embodiment, the at least one polypeptide is encoded by a nucleotide sequence originating from the genome of a pathogen, such as a virus, especially a retrovirus, lentivirus, flavivirus or coronavirus, of a bacterium or of a parasite. The expression "*originating from"* means that the nucleotide sequence of the polynucleotide has a counterpart in the pathogen genome. In the particular case of viruses, the viral genome may be, either in the native form contained in the virus (same nature of the genome) or in a different form (different nature of the genome). For example, if the polynucleotide is provided in the lentiviral vector for use according to the invention under the form of a double stranded DNA whereas in the corresponding native viral genome, it is naturally present in the form of a single strand RNA, the fragment of the viral genome having the nucleotide sequence of the polynucleotide is retrotranscribed to provide a double stranded fragment. In another example, if the polynucleotide is provided in the lentiviral vector for use according to the invention under the form of a positive RNA whereas the viral genome is naturally present in the form of a negative RNA, the fragment of the viral genome is "replicated" to provide the complementary strand.

In another embodiment, and either independently of or in combination with the above-embodiments of the invention, at least one polypeptide comprises or consists in surface antigens, such as viral envelope or other membrane proteins, and fragments thereof, for example envelope from AIDS viruses, including HIV-1 or HIV-2, or for example envelope from the Yellow Fever Virus, the West Nile Virus, the Dengue virus (DV), the Japanese encephalitis virus (JEV) or the SARS-associated coronavirus. Other interesting viral polypeptides are from the capsid of HIV.

In a particular aspect, the at least one polypeptide is encoded by a polynucleotide of lentiviral origin (for example from *gag* or *pol,* or for example from *env*)*.* In a particular aspect, said coding polynucleotides are not the complete *gag* or *pol* gene or not the complete *env* gene, or are not a functional version of these genes *i.e*., a gene encoding a functional protein. For example, they have a size ranging from 30 to 500 bp, preferably 30 to 300 bp, more preferably 30 to 100 bp.

In another aspect, the polynucleotide encoding said at least one polypeptide does not originate from a lentivirus genome (hence it is a heterologous sequence with respect to the vector genome).

Alternatively, the at least one polypeptide is derived from a tumoral antigen or a tumoral epitope. Particular polypeptides (or part thereof) are those expressed on the cell surface of tumoral cells. These polypeptides (or part thereof) may originate from the cell (self peptide) either in a wild type or mutated form; they also may originate from a virus that transforms a normal cell in tumor cell (tumor virus). Examples of such viruses, etiologic agents for human cancer, are the Human Papilloma Virus (HPV) causing cervical cancer, the Epstein-Barr Virus causing lymphoma through the EBV-induced membrane antigen (EBMA), HTLV-1 causing Acute T cell leukaemia (ACT) through the HTLV-1 tax protein, the human herpes virus type 8 (HHV8), the hepatitis B virus (HBV) and the hepatitis C virus (HCV).

Finally said at least one polypeptide is an artificial (non-natural) polypeptide, preferably a multiepitope polypeptide. This multiepitope polypeptide encodes at least two epitopes, originating from a pathogenic organism, including viruses, and/or of tumoral-origin. In a particular aspect, said at least two epitopes originate from the same virus or from the same tumor cell; in that case, said at least two epitopes may be selected for their different MHC (HLA) restriction. In another aspect, said at least two epitopes originate from different viruses, or from different tumor cells. Said epitopes can be arranged consecutively, *i.e*., the 3' end of the epitope is directly linked to the 5' end of the second epitope (and so on), corresponding to a polynucleotide encoding a peptide sequence exclusively composed of consecutive epitopes. The at least two epitopes can alternatively be separated by a one-amino acid spacer or a peptide spacer *i.e.*, meaning that the different polynucleotide units are separated by one or several codon(s) encoding respectively one or several amino acid(s). As spacers improving the processing of multiple epitopes, 4 amino acid-peptides composed of an arginine (R) in the C terminal position and hydrophilic residues (A, K, D and/or T) in other positions are preferred. Especially, 4 amino acid-peptides having a positively charged residue or an acidic residue in the C terminal position may be used, dependently or independently of hydrophilic residues (A, K, D and/or T) in other positions. In a particular aspect, said spacers are internal processing sequences such as endosomal or lysosomal processing sequences, enabling the better processing of the multiple epitopes and avoiding the processing of new peptides resulting from overlapping cutting. Such a separation having recourse to a spacer can be used to separate all or part of the epitopes.

The expression "*immunogenic composition*" refers to a composition comprising at least the lentiviral vector for use according to the invention as active principle, said composition being suitable for administration into a host. This composition may comprise further a pharmaceutically suitable excipient or carrier and/or vehicle, when used for systemic or local administration. A "*pharmaceutically acceptable carrier*" refers to a non-toxic solid, semisolid or liquid filler, diluent, encapsulating material or formulation auxiliary of any conventional type. A *"pharmaceutically acceptable carrier*" is non-toxic to recipients at the dosages and concentrations employed and is compatible with other ingredients of the formulation; Suitable carriers include, but are not limited to, phosphate buffered saline solutions, distilled water, emulsions such as an oil/water emulsions, various types of wetting agents sterile solutions and the like, dextrose, glycerol, saline, ethanol, and combinations thereof.

The immunogenic composition for use according to the invention has the capacity, despite the absence of integration of the transgene into the genome of the host cell, to elicit an immune response *i.e*., any reaction by the immune system of the host against said at least one polypeptide (encoded by said transgene).

The immune response can be a humoral response *i.e*., antibodies, elicited by said composition, are produced against said at least one polypeptide of the lentiviral vector. Said humoral response is a protective humoral response. The protective humoral response results mainly in maturated antibodies, having a high affinity for their antigen, such as IgG. In a particular aspect, the protective humoral response is T-cell dependent. In a particular embodiment, the protective humoral response induces the production of neutralizing antibodies.

The immune response can be a cellular immune response (T-cell immune response), particularly a CD8-mediated cellular immune response or a CD4-mediated cellular immune response *i.e*., an immune response which is mediated by activated cells harbouring CD8 or CD4 receptors, preferably Cytotoxic T lymphocytes (CTL).

In a particular embodiment of the invention, the lentiviral vector despite the defective integrase, is able to elicit an early immune response. The expression "*early immune response"* refers to a protective immune response (protection against the pathogen or tumoral cell bearing said at least one polypeptide) that is conferred within about one week after the administration of the composition.

In another embodiment, the immune response conferred by the composition is a long lasting immune response *i.e*., said immune response can be still detected at least two months, preferably at least 3 months and most preferably at least 6 months after the administration of the composition. When the immune response is humoral, the long lasting response can be shown by the detection of specific antibodies, by any suitable methods such as ELISA, immunofluorescence (IFA), focus reduction neutralization tests (FRNT), immunoprecipitation, or Western blotting.

In another embodiment, independent of the above-embodiment, the strength of the immune response conferred by the composition for use according to the invention is dependent upon the injected doses of the lentiviral vectors; the higher the dose, the higher the immune response strength.

Interestingly, said immune response, either humoral or cellular, early immune response and/or long lasting immune response, is elicited after a single administration of the composition.

The compositions quoted above can be injected in a host via different routes: subcutaneous (s.c.), intradermal (i.d.), intramuscular (i.m.) or intravenous (i.v.) injection, oral administration and mucosal administration, especially intranasal administration or inhalation. The quantity to be administered (dosage) depends on the subject to be treated, including considering the condition of the patient, the state of the individual's immune system, the route of administration and the size of the host. Suitable dosages range from 1 to 100 µg, preferably 1 to 50 µg and most preferably 1 to 10 µg, and can be modified by one skilled in the art, depending on circumstances. When formulated for subcutaneous injection, the immunogenic composition preferably comprises between 1 and 100 µg of the lentiviral vector per body weight of the host, more preferably from 1 to 30 µg/dose, especially around 10 µg/dose, in a sole injection. The dose corresponds to p24 antigen content.

The term "*host*" as used herein means any mammal encompassing either non-human, including primate, mouse, rat, pets or laboratory animals or rather a human being. In the context of the present invention, the host is a human being.

Therefore, the lentiviral vector for use according to the invention once administrated into the human being, infects cells of the human being, possibly specific cells, depending on the envelope proteins it was pseudotyped with. The infection leads to the release of the lentiviral genome into the cytoplasm of the human cell where the retrotranscription takes place. Once under a triplex form (via the DNA flap), the lentiviral genome is imported into the nucleus, where the transgene is expressed via the cellular machinery. The expression is temporary in absence of origin of replication in the lentiviral genome, because of nucleic acid degradation and cell division. The expression may be longer by providing an origin of replication ensuring a proper diffusion of the lentiviral genome into daughter cells after cell division. The stability may also be increased by insertion of MAR or SAR elements.

Despite the absence of integration of the lentiviral genome into the human cell genome, the at least one polypeptide encoded by the transgene is sufficiently expressed and longer enough to be processed, associated with MHC molecules and finally directed towards the cell surface. Depending on the nature of the transgene, the at least one polypeptide epitope associated with the MHC molecule triggers a humoral or cellular immune response.

The lentiviral vectors for use according to the invention are useful for the prophylaxis of pathogen infection as well as malignant states, in a host.

The expression "*prophylaxis of pathogen infection*" encompasses the use of a lentiviral vector to avoid the apparition of symptoms after the contamination or a suspicion of contamination, and/or to prevent or to reduce the infection of the host by said pathogen, particularly by a virus.

The expression "*malignant states"* refers to uncontrolled cell proliferation, including tumors, resulting from the altered gene-expression pattern of a cell or a group of cells, or resulting from the transformation of a normal cell(s) by a tumor virus, as well as the detrimental consequences accompanying such tumors *e*.*g*., cancer. The "*prophylaxis of malignant states"* encompasses preventing the uncontrolled cell proliferation, the establishment of tumoral cells, or the infection or transformation of cells by a tumor virus.

The term treatment encompasses the curative effect achieved with lentiviral vectors (destruction of the pathogen or the tumor cells) and also the beneficial effect for the host undergoing the treatment, said effect being either obtained at cellular level or clinical level, including as a result, an improvement of the condition of the host and/or a remission state or a recovery of a health state. In pathogen infection, "*treatment*" encompasses also the complete elimination of the pathogen, or the impossibility of detecting said pathogen by conventional methods, but also the decrease of the pathogen in the host or the improvement of the clinical condition of the patient. Referring to malignant states, "*treatment*" further encompasses the decrease in the size and/or number of tumors in the host or the improvement of the clinical condition of the patient.

In a particular aspect, the composition for use according to the invention further comprises additional active compounds useful for the prophylaxis or the treatment of pathogen infection or tumors, either general compounds or compounds proved to be active in a pathogen-specific infection or tissue-specific cancer.

The lentiviral vector for use according to the invention is obtainable by a transcomplementation system (vector/packaging system) by transfecting *in vitro* a permissive cell (such as 293T cells) with a plasmid containing the lentiviral vector genome and at least one other plasmid providing, *in trans,* the *gag, pol* and *env* sequences encoding the polypeptides GAG, POL and the envelope protein(s), or for a portion of these polypeptides sufficient to enable formation of retroviral particles. As an example, permissive cells are transfected with a first plasmid comprising the lentiviral vector genome (transfer vector), a second plasmid (envelope expression plasmid or pseudotyping env plasmid) comprising a gene encoding an envelope protein(s) (such as VSV-G), and a third plasmid (encapsidation plasmid or packaging construct) expressing the GAG and POL proteins.

The present description also discloses with the same definitions as the ones given above, a lentiviral vector comprising a defective integrase protein and further comprising a polynucleotide encoding at least one antigenic polypeptide, to produce an immunogenic composition for use in the elicitation of an immune response against said at least one polypeptide, in a host in need thereof, as well as a method to elicit an immune response against at least one polypeptide, in a host in need thereof, comprising administrating in said host a lentiviral vector or a composition comprising said lentiviral vector, wherein said vector comprises a defective integrase protein and further comprises a polynucleotide encoding said at least one antigenic polypeptide.

### MATERIALS AND METHODS

### Cell culture and virus preparations

Hela cells (ATCC CCL-2), Human 293T cells and African green monkey kidney Vero cells (ATCC CCL-81) were cultured in Dulbecco modified Eagle medium (DMEM) supplemented with 10% (Hela cells, 293T cells) or 5% (Vero cells) heat-inactivated fetal calf serum (FCS), penicillin, streptomycin and Glutamax (GIBCO). West Nile Virus (WNV) strain IS-98-ST1 (GenBank accession number AF 481 864), a closely related variant of NY99 strain¹⁰, was propagated in mosquito *Aedes pseudoscutellaris* AP61 cell monolayers. Purification in sucrose gradients and virus titration on AP61 cells (*Aedes pseudoscutellaris* cells) by focus immunodetection assay (FIA) using anti-WNV hyperimmune mouse ascitic fluid (HMAF) were performed as previously described. Infectivity titers were expressed as focus forming units (FFU).

### Lentiviral vector production

The TRIP_{sEWNV} (Figure 7) and TRIP_{GFP} vector plasmids were constructed as previously described (Iglesias et al. J. Gene Med. 2006 Mar; 8(3): 265-74). The nucleotide sequences of these two vectors are presented respectively on Figures 8 and 9. Vector particles were produced by transient calcium phosphate co-transfection of 293T cells with the vector plasmid pTRIP_{sEwnv} or pTRIP_{GFP}, a VSV-G envelope expression plasmid (pHCMV-G) and an encapsidation plasmid (p8.74 or pD64V for the production of integration-proficient or integration-deficient vectors respectively) as previously described. Quantification of the p24 antigen content of concentrated vector particles was performed with a commercial HIV-1 p24 enzyme-linked immunosorbent assay (ELISA) kit (Perkin Elemer Life Sciences).

### Preparation of bone marrow-derived DCs

Bone marrow cells were isolated by flushing mice femurs and tibiae with RPMI supplemented with 10% FCS. Cells were then passed through a 45-µm cell strainer, centrifuged and resuspended in IOTest® 3 lysing solution (an erythrocyte lysing solution, mixture of ammonium chloride, potassium bicarbonate and ethylenediamine tetraacetic acid (EDTA); Beckman Coulter) and incubated at 4°C for 5 min to lyze red blood cells. The cells were centrifuged and cultured for 8 days at 1x10⁶ cells/ml in culture medium consisting of RPMI with 10% FCS, L-glutamine, penicillin, streptomycin, 1 mM sodium pyruvate, 10 mM HEPES, and 50 µM 2-mercaptoethanol supplemented with 100 ng/ml of recombinant mouse FLT3 ligand (R&D Systems).

### Transduction experiments and flow cytometry analysis

For transduction experiments on non-dividing cells, Hela cells were seeded in 48 wells plates at 40,000 cells/well in the presence of 8 µM of aphidicolin (Sigma). Cells were transduced with lentiviral vectors at a concentration ranging from 1 to 100 ng/ml, 24 hours after the aphidicolin block, which was replenished in the medium at the time of transduction. At 2 days post-transduction, cells were harvested and eGFP expression was analyzed by flow cytometry.

For DC transduction experiments, 500,000 FLT3L-generated-bone marrow-derived DC (FL-DC) were transduced at day 6 of the differentiation, with lentiviral vectors at a concentration ranging from 50 to 300 ng/ml. At 2 days post-transduction, FL-DC were harvested and resuspended in PBS with 2% FCS and 0.01% sodium azide (staining buffer). Cells were strained with an APC(allophycocyanine)-conjugated anti-CD11c antibody and a PerCP(Peridinin chlorophyll protein)-conjugated anti-B220 antibody, washed twice and analyzed by flow cytometry on a FACSCalibur (BD biosciences, Franklin Lakes, NJ).

### Mice immunization

All animal experiments were conducted in accordance with the guidelines of the Office Laboratory of Animal Care at the Pasteur Institute. Six-week-old C57/B16 mice were intraperitoneally (i.p.) inoculated with varying doses of TRIP/sE WNV vector particles (from 1 to 100 ng/ml) in 0.1 ml Dulbecco's phosphate-buffered saline (DPBS; pH 7.5) supplemented with buffered 0.2% bovine serum albumin (DPBS/0.2% BSA, Sigma).

### Measurement of serum antibody responses

Mice were bled via the periorbital route and serum samples were heat-inactivated 30 min at 56°C. Anti-WNV antibodies were detected by ELISA, by use of microtitration plates coated with sucrose-purified WNV IS-98-ST1. Peroxydase goat anti-mouse immunoglobulin (H+L) (Jackson Immuno Research) was used at a 1:4,000 dilution as secondary antibodies. The endpoint titer was calculayed as the reciprocal of the last dilution eliciting twice the optical density (OD) of sera from nonimmunized mice.

### WNV challenge

WNV challenge was performed by i.p. inoculation of neurovirulent WNV strain IS-98-ST1 (i.p. LD 50 = 10 FFU) as previously described, either one week or two months after lentiviral vector vaccination. The challenged mice were monitored daily for signs of morbidity or mortality, for up to 21 days after the WNV strain inoculation.

### RESULTS

### Transduction of nondividing cells with TRIP vectors deficient for integration results in high transgene expression levels

To test the hypothesis that integration deficient LV (TRIP.NI vectors) could be efficient tools to deliver antigen (Ags) to nondividing APC such as DC, we initially evaluated their transduction efficiency of growth-arrested cells. For this purpose, HeLa cells treated with aphidicolin, a specific inhibitor of cell cycle, were exposed to graded doses of TRIP.NI or TRIP.I particles encoding eGFP. The transduction efficiency was then determined by flow cytometry. As shown in Figure 1 (upper panel), TRIP.NI vectors transduced nondividing cells with high efficiency and in a dose dependent manner. Moreover, analysis of the percent of eGFP positive cells revealed marginal differences in the capacities of transduction of TRIP.NI vectors compared to that of TRIP.I vectors. Transduction with TRIP.NI particles yielded also high levels of expression of the transgene (Figure 1, lower panel), although significantly lower by a 2-fold factor compared to TRIP.I-transduced cells.

### TRIP nonintegrative lentiviral vector transduction leads to effective antigen expression both in conventional and in plasmacytoid dendritic cells

We next studied the ability of TRIP.NI vectors to transduce DC. DC are categorized as conventional (cDC) (CD11c⁺B220⁻) and plasmacytoid (pDC) (CD11c⁺B220⁺) and both these DC subtypes are able to stimulate Ag-specific immune responses. We then investigated the transduction of bone marrow-derived DC differentiated in presence of Flt3L (FL-DC), which allows the generation of large numbers of pDC and cDC. FL-DC were exposed to graded doses of TRIP.NI_{GFP} or TRIP.I_{GFP} particles. As shown in Figure 2A, both TRIP.I and TRIP.NI vectors were capable of transducing FL-DC with maximal transduction of efficiency of 60% and 56% respectively. Interestingly, we observed that transduction with TRIP.I particles led to a small proportion of DC expressing high levels of eGFP whereas transduction experiments with TRIP.NI did not (see the presence of dots in the right top corner of the dot blot, in experiments where cells have been transduced with the lentiviral vectors of the invention as compared to HI vectors),. To rule out the possibility of pseudo-transduction conferred by residual eGFP proteins contaminating the vector stock, we also evaluated the percentage of transduced DC after exposure to particles submitted prior to a heat-treatment, which has been shown to abrogate the transduction capabilities of LV on different cell types. As expected, the heat-treatment decreased drastically the percentage of eGFP positive cells (Figure 2A).

We next gated on CD11c⁺B220⁺ dendritic cells and CD11c⁺B220⁻ dendritic cells to evaluate the capacity of LV to transduce each DC subset. As shown in Figure 2B, not only FL-derived cDC but also FL-derived pDC could be efficiently transduced with LV, regardless of their integration proficiencies.

Transduction efficiency with TRIP.NI particles was dose dependent and slightly but insignificantly lower than those obtained with TRIP.I particles. Interestingly, we observed that transduction with TRIP.I vectors led to a small proportion of DC expressing high levels of the transgene, whereas exposure of DC to TRIP.NI vectors did not (Fig. 2A). This cellular population which was only observed in transduction experiments with TRIP.I vectors could be the consequence of multiple-vector integrations or integration of the vector in active transcription regions of the genome.

### TRIP nonintegrative lentiviral vectors induce the production of Ag-specific antibodies

Taking into account that TRIP.NI could efficiently deliver a foreign gene to DC, we next explored their ability to mount a specific immune response. In a recent study, we have designed TRIP.I vectors coding for a secreted form of WNV envelope (TRIP.I E_{WNV}) which possesses neutralizing epitopes and we have demonstrated that TRIP.I E_{WNV} could stimulate an antibody-based protective immunity in a mouse model of WNV infection. To investigate the ability of TRIP.NI vectors to initiate a B cell response, animals were immunized with various doses of TRIP.NI E_{WNV} particles ranging from 1 to 100 ng of p24 antigen per mouse. As a control, mice were inoculated with 100ng of TRIP.NI E_{WNV} particles inactivated by heating (HI) to abrogate their transduction capacities. Three weeks after immunization, mice were bled periorbitally and individual or pooled sera were tested by ELISA for anti-WNV total antibodies. As expected, immunizations with heat-inactivated TRIP.NI E_{WNV} vectors were not followed by the production of Abs (Fig.3A). By contrast, mice immunized with a dose as low as 10 ng of TRIP.NI E_{WNV} vectors displayed detectable levels of anti-WNV antibodies and immunizations with 100 ng of sE-NILV induced a massive secretion of anti-WNV Ig with a mean titer reaching 8 x 10⁴.

We next compared the strength of the immune response elicited by TRIP.NI E_{WNV} and TRIP.I E_{WNV} vectors. As shown in Fig. 3B, vaccination with TRIP.I E_{WNV} at a dose as low as 3ng of particles generated a very high secretion of anti-WNV antibodies and titers were relatively constant within the range of immunizing doses from 3 to 100ng, with no dose response evident. By contrast and contrary to all expectations, titers in sera from mice immunized with TRIP.NI E_{WNV} vectors were proportional to the dose of particles injected. Although TRIP.I vectors elicited a higher immune response than TRIP.NI vectors at doses below 30ng, vaccinations with 100 ng of either vectors led to an equivalent response.

Taken together, these results demonstrated that a single immunization with TRIP.NI vectors was sufficient to elicit a humoral specific immune response with a strength comparable to that obtained with TRIP.I vectors, above a threshold dose of particles. Interestingly and surprisingly, use of non-integrative vectors enable to obtain an immune response whose strength is dependent upon the dose of injected lentiviral vectors.

Immunizations of mice with a single dose of TRIP.NisEwnv give the following antibody titers:

| **Dose** | **WNV specific antibody titer (O.D.)** |
|---|---|
| HI NI 100 | 0 |
| NI 1 | 0 |
| NI 3 | 0 |
| NI 10 | 152 |
| NI 30 | 569 |
| NI 100 | 83000 |

As shown on Figure 3A, a potent secretion of specific WNV antibodies, with a mean titer reaching 8 x 10⁴ at a dose of 100ng of p24 antigen is obtained. At this dose, immunizations with TRIP.NI led to an equivalent response to that obtained with TRIP.I. However, dose-response experiments revealed that the minimal dose required for the induction of a B cell response was lower with TRIP.I particles compared to the TRIP.NI particles. One possible explanation for this result could be related to the ability of TRIP.I vectors to generate Ag-highly-expressing DC since, on theorical grounds, high expression levels of the Ag in the DC could favor a more sustained presentation of antigenic peptides and thus may explain why low doses of TRIP.I particles were sufficient to elicit a specific immune response. This hypothesis may also explain the non-linearity of the WNV antibody production observed in dose-response immunization experiments with TRIP.I vectors (Fig.3B). Indeed, the *in vitro* dose response experiments performed on DC revealed that the appearance of Ag-highly-expressing DC do not seem to be correlated to the dose of TRIP.I particle (Fig.2A). Thus, the capacity to generate Ag-highly-expressing DC may contribute to explain the differences observed between TRIP.I and TRIP.NI with low doses of particles injected. Another possibility is linked to the fact that VSV-G pseudotyped LV have a large cellular tropism and thus, may transduce at the site of injection other cell types than DC, including dividing cells. This could result in a more sustained expression of the Ag in vaccination experiments with TRIP.I particles. Which cell types are transduced after in vivo injections of LV and to what extend they are involved in the magnitude of the immune response elicited by TRIP.I and TRIP.NI vectors is the subject of ongoing research.

### Immunization with TRIP.NI E_{WNV} vectors confers early protection against WNV challenge

We have previously shown that TRIP.I E_{WNV} confers an early protective immunity against a WNV challenge. To determine if the immune response elicited by TRIP.NI vectors could also lead to a rapid protection, mice were immunized with 100 ng of TRIP.NI E_{WNV} particles and challenged 7 days after with 10,000 FFU of the highly virulent WNV strain IS-98-ST1 (thousand times the dose required to kill 50% of infected animals). We included also in this challenge experiment a group of mice immunized with 100 ng of TRIP.I E_{WNV} as a positive control of protection and another group of mice inoculated with D-PBS as a negative control. As expected, all mice that received D-PBS died within 12 days post-challenge (Fig. 4). In contrast, all mice immunized with a single dose of TRIP.NI E_{WNV} were protected from the challenge, as were mice immunized with TRIP.I E_{WNV}. Mice protected from WNV challenge did not develop clinical signs of illness during the 3-weeks post-challenge observation period. These results demonstrated that an early protective immunity against WNV was achieved with a single administration of TRIP. E_{WNV} defective for integration.

### TRIP.NI E_{WNV} induces long-lasting protection

As demonstrated earlier, immunization of mice with TRIP.I E_{WNV} resulted in the establishment of long-term protective immunity against WNV challenge. To evaluate the duration of the protective immunity elicited by TRIP.NI E_{WNV}, and the minimum dose of particles required to induce long-term protection, mice were immunized with different amounts of particles (1, 3, 10, 30 and 100 ng of p24 antigen) and were challenged after 2-month waiting period after immunization. As shown in Figure 1A, there was a dose-dependent relationship between the dose of TRIP.NI E_{WNV} particles administrated and the degree of protection, with a fully protection achieved at a dose of 100 ng of vaccine particles injected.

Thus, TRIP.NI E_{WNV} vectors induced long-lasting immunity against WNV infection

### DISCUSSION

An important result of the present experiments is the demonstration that vaccination with TRIP.NI particles can provide an efficient and strong immune response that is both an early and long lasting immune response, and further antigen dose-dependent, despite the absence of integration of the lentiviral genome administered. Therefore, a fully protection against a challenge with a lethal dose of WNV was demonstrated.

As expected, memory protective immunity was directly correlated to the titer of anti WNV antibodies induced by TRIP.NI particles (Fig. 3 and Fig. 5). Indeed, it is well established that humoral immunity is a critical component for the establishment of a fully protective immunity against WNV, as specific antibodies limit dissemination of the infection. Intriguingly, heat-inactivated TRIP.NI particles as well as HI-TRIP.I particles were able to confer a partial protection (30%) against WNV challenge (data not shown), although no WNV-antibodies were detected in the sera of animals 3 weeks after injection of HI-TRIP particles (Fig. 3A, B). This suggests that cellular immunity could also play a partial role in the establishment of protection against WNV. Consistent with this hypothesis, mice that lack CD8⁺ cells have increased mortality after WNV infection (Shoresta and Diamonds, unpublished data). Moreover, cytotoxic T cell epitopes have been defined in the domain III of the envelope of several flaviviruses. Additional works are required to clarify the relative contribution of CTL responses to the long term protection conferred by TRIP.NI and TRIP.I vaccines. Moreover, further studies are also needed to define the molecular mechanisms allowing the entry of HI-TRIP particles in DC since the heat-treatment denatures the VSV-G envelope and has been shown to abrogate the transduction capacities of LV in different cell lines. However, it is tempting to speculate that, in regards to the exceptional internalization capacities of DC, a fraction of HI TRIP particles could be incorporated in DC by a VSV-G independent mechanism, allowing a low but sufficient Ag expression to explain the partial protection conferred by HI-TRIP particles.

Kinetic challenge experiments on vaccinated mice revealed that TRIP.NI vaccines not only conferred a long term protective immunity but elicited also protection as early as one week after a single injection of particles. Although the exact mechanisms involved in this early protection are not fully understood, we have detected specific WNV antibodies one week after immunizations with TRIP.NI and TRIP.I particles. We have previously shown that this early wave of antibodies were exclusively composed of specific IgM, derived from mice 4 days after injection, completely protected mice against WNV infection.

In our study, a vaccination regimen based on a direct injection of a single dose of TRIP.NI particles elicited a robust, rapid and long term specific immune response, achieving fully protection against WNV. Thus, TRIP.NI based vaccine strategy represents a safe and efficacious platform for the development of vaccines against pathogens agents such as flaviviruses that require B cell immunity.

## Claims

1. Use of a non-integrating HIV lentiviral vector particle comprising a defective integrase protein and further comprising a RNA genome which is the transcription product of a vector genome comprising a polynucleotide encoding at least one antigenic polypeptide, two LTRs and a DNA Flap comprising a cPPT and a CTS domains, in the manufacture of an immunogenic composition for eliciting a protective immune response against said at least one antigenic polypeptide to prevent infection by a pathogen or malignant states, in a human being administered with said immunogenic composition comprising doses of said HIV lentiviral vector particles ranging from 1 µg to 100 µg of p24 antigen, wherein said defective integrase protein is an integrase having a class I mutation to specifically alter its integrase activity.

2. Use of a HIV lentiviral vector particle according to claim 1, wherein said lentivirus vector is non-replicative.

3. Use of a HIV lentiviral vector particle according to any one of claims 1 to 2, wherein said immune response is a protective humoral immune response.

4. Use of a HIV lentiviral vector particle according to any one of claims 1 to 2, wherein said immune response is a cellular immune response.

5. Use of a HIV lentiviral vector particle according to claim 4, wherein said immune response is a CD8-mediated cellular immune response.

6. Use of a HIV lentiviral vector particle according to claim 4, wherein said immune response is a CD4-mediated cellular immune response.

7. Use of a HIV lentiviral vector particle according to claim 1, wherein said mutation is D64V.

8. Use of a HIV lentiviral vector particle according to any one of claims 1 to 7, wherein the lentiviral genome is devoid of gag, pol and/or env lentiviral genes.

9. Use of a HIV lentiviral vector particle according to any one of claims 1 to 7, wherein the lentiviral genome is devoid of functional gag, pol and/or env lentiviral genes.

10. Use of a HIV lentiviral vector particle according to any one of claims 1 to 8, wherein the lentiviral genome is devoid of all endogenous coding lentiviral sequences.

11. Use of a HIV lentiviral vector particle according to any one of claims 1 to 10, wherein the lentiviral genome comprises (a) a LTR5' and a LTR3', (b) a psi sequence, (c) a DNA flap comprising a cPPT and a CTS domains and (d) a sequence encoding said at least one polypeptide.

12. Use of a HIV lentiviral vector particle according to claim 11, wherein said lentiviral genome further comprises a splice donor site (SD), a splice acceptor site (SA) and a Rev-responsive element (RRE).

13. Use of a HIV lentiviral vector particle according to claim 11 to12, wherein said lentiviral genome further comprises an origin or replication (ori).

14. Use of a HIV lentiviral vector particle according to any one of claims 11 to 13, wherein said lentiviral genome further comprises a scaffold attachment region (SAR) and/or a matrix attachment region (MAR).

15. Use of a HIV lentiviral vector particle according to any one of claims 1 to 14, wherein the LTR is deleted for the promoter and the enhancer of U3.

16. Use of a HIV lentiviral vector particle according to any one of claims 1 to 15, wherein the DNA flap comprises or consists in the sequences as defined in SEQ ID NO: 1 to 7, as depicted in Figure 6.

17. Use of a HIV lentiviral vector particle according to any one of claims 1 to 16, wherein said at least one polypeptide is encoded by a sequence originated from the genome of a pathogen.

18. Use of a HIV lentiviral vector particle according to claim 17, wherein said pathogen is a virus, especially retrovirus, lentivirus, flavivirus or coronavirus, a bacterium or a parasite.

19. Use of a HIV lentiviral vector particle according to claim 18, wherein said at least one polypeptide is derived from the envelope of AIDS viruses, including HIV-1 or HIV-2, from capsid of HIV or from envelope of the Yellow Fever Virus, the West Nile Virus, the Dengue virus (DV), the Japanese encephalitis virus (JEV) or the SARS-associated coronavirus.

20. Use of a HIV lentiviral vector particle according to any one of claims 1 to 16, wherein said at least one polypeptide comprises a tumoral epitope or antigen.

21. Use of a HIV lentiviral vector particle according to any one of claims 1 to 20, wherein said lentiviral vector is pseudotyped with a VSV-G protein.

22. Use of a HIV lentiviral vector particle according to any one of claims 1 to 21, wherein said VSV-G protein comprises or consists of the transmembrane domain of the Indiana VSV-G and the ectodomain of the New-Jersey serotype.

23. Use of a HIV lentiviral vector particle according to any one of claims 1 to 22, wherein said immune response is an early immune response.

24. Use of a HIV lentiviral vector particle according to claim 23, wherein said immune response is induced as short as one week after the administration of the composition.

25. Use of a HIV lentiviral vector particle according to any one of claims 1 to 24, wherein said immune response is a long lasting immune response.

26. Use of a HIV lentiviral vector particle according to claim 25, wherein said immune response is still protective at least two months after the administration of the composition.

27. Use of a HIV lentiviral vector particle according to any one of claims 1 to 26, wherein said immune response in said host is reached with a single administration.

28. Use of a HIV lentiviral vector particle according to any one of claims 1 to 27, which is administered in doses of said HIV lentiviral vector ranging from 1 to 50µg, more preferably 1 to 10 µg.

29. Use of a HIV lentiviral vector particle according to any one of claims 1 to 28, wherein said composition is administered by a subcutaneous (s.c.), intradermal (i.d.), intramuscular (i.m.) or intravenous (i.v.) injection, an oral administration and a mucosal administration, especially intranasal administration or inhalation.

30. Use of a HIV lentiviral vector particle according to any one of claims 1 to 29, to prevent infection of the host by a pathogen such as a virus, a bacterium or a parasite.

## Patentansprüche

1. Verwendung eines nicht integrierenden HIV-lentiviralen Vektorpartikels umfassend ein defektes Integraseprotein und weiterhin umfassend ein RNA-Genom, das das Transkriptionsprodukt eines Vektorgenoms ist, das ein Polynukleotid umfasst, das mindestens ein antigenes Polypeptid, zwei LTRs und eine DNA-Flap, die eine cPPT- und eine CTS-Domäne umfasst, kodiert, bei der Herstellung einer immunogenen Zusammensetzung zum Hervorrufen einer protektiven Immunantwort gegen das mindestens eine antigene Polypeptid, um Infektion durch ein Pathogen oder bösartigen Zuständen vorzubeugen, in einem Menschen, dem die immunogene Zusammensetzung verabreicht wurde, die Dosen der HIV-lentiviralen Vektorpartikel umfasst, die sich im Bereich von 1 µg bis 100 µg an p24-Antigen befinden, wobei das defekte Integraseprotein eine Integrase mit einer Klasse I-Mutation ist, um spezifisch ihre Integraseaktivität zu verändern.

2. Verwendung eines HIV-lentiviralen Vektorpartikels nach Anspruch 1, wobei der Lentivirusvektor nicht replikativ ist.

3. Verwendung eines HIV-lentiviralen Vektorpartikels nach einem beliebigen der Ansprüche 1 bis 2, wobei die Immunantwort eine protektive humorale Immunantwort ist.

4. Verwendung eines HIV-lentiviralen Vektorpartikels nach einem beliebigen der Ansprüche 1 bis 2, wobei die Immunantwort eine zelluläre Immunantwort ist.

5. Verwendung eines HIV-lentiviralen Vektorpartikels nach Anspruch 4, wobei die Immunantwort eine CD8-vermittelte zelluläre Immunantwort ist.

6. Verwendung eines HIV-lentiviralen Vektorpartikels nach Anspruch 4, wobei die Immunantwort eine CD4-vermittelte zelluläre Immunantwort ist.

7. Verwendung eines HIV-lentiviralen Vektorpartikels nach Anspruch 1, wobei die Mutation D64V ist.

8. Verwendung eines HIV-lentiviralen Vektorpartikels nach einem beliebigen der Ansprüche 1 bis 7, wobei dem lentiviralen Genom gag-, pol- und/oder envlentivirale Gene fehlen.

9. Verwendung eines HIV-lentiviralen Vektorpartikels nach einem beliebigen der Ansprüche 1 bis 7, wobei dem lentiviralen Genom funktionale gag-, pol- und/oder env-lentivirale Gene fehlen.

10. Verwendung eines HIV-lentiviralen Vektorpartikels nach einem beliebigen der Ansprüche 1 bis 8, wobei dem lentiviralen Genom alle endogenen kodierenden lentiviralen Sequenzen fehlen.

11. Verwendung eines HIV-lentiviralen Vektorpartikels nach einem beliebigen der Ansprüche 1 bis 10, wobei das lentivirale Genom (a) eine LTR5' und eine LTR3', (b) eine psi-Sequenz, (c) eine DNA-Flap, die eine cPPT- und eine CTS-Domäne umfasst und (d) eine Sequenz, die das mindestens eine Polypeptid kodiert, umfasst.

12. Verwendung eines HIV-lentiviralen Vektorpartikels nach Anspruch 11, wobei das lentivirale Genom weiterhin eine Splice-Donorstelle (SD), eine Splice-Akzeptorstelle (SA) und ein Rev-responsives Element (RRE) umfasst.

13. Verwendung eines HIV-lentiviralen Vektorpartikels nach Anspruch 11 bis 12, wobei das lentivirale Genom weiterhin einen Replikationsursprung (ori) umfasst.

14. Verwendung eines HIV-lentiviralen Vektorpartikels nach einem beliebigen der Ansprüche 11 bis 13, wobei das lentivirale Genom weiterhin eine Kerngerüst-Anheftungsregion (*scaffold attachment region,* SAR) und/oder eine Kernmatrix-Anheftungsregion (*matrix attachment region,* MAR) umfasst.

15. Verwendung eines HIV-lentiviralen Vektorpartikels nach einem beliebigen der Ansprüche 1 bis 14, wobei die LTR für den Promotor und den Enhancer von U3 deletiert ist.

16. Verwendung eines HIV-lentiviralen Vektorpartikels nach einem beliebigen der Ansprüche 1 bis 15, wobei die DNA-Flap wie in Figur 6 abgebildet SEQ ID NO: 1 bis 7 umfasst oder aus ihnen besteht.

17. Verwendung eines HIV-lentiviralen Vektorpartikels nach einem beliebigen der Ansprüche 1 bis 16, wobei das mindestens eine Polypeptid durch eine Sequenz kodiert ist, die aus dem Genom eines Pathogens stammt.

18. Verwendung eines HIV-lentiviralen Vektorpartikels nach Anspruch 17, wobei das Pathogen ein Virus, insbesondere Retrovirus, Lentivirus, Flavivirus oder Coronavirus, ein Bakterium oder ein Parasit ist.

19. Verwendung eines HIV-lentiviralen Vektorpartikels nach Anspruch 18, wobei das mindestens eine Polypeptid aus der Hülle von AIDS-Viren, einschließlich HIV-1 oder HIV-2, aus dem Kapsid von HIV oder aus der Hülle des Gelbfiebervirus, des West-Nil-Virus, des Dengue-Virus (DV), des Japanische Enzephalitis-Virus (JEV) oder des SARS-assoziierten Coronavirus abgeleitet ist.

20. Verwendung eines HIV-lentiviralen Vektorpartikels nach einem beliebigen der Ansprüche 1 bis 16, wobei das mindestens eine Polypeptid ein Tumorepitop oder Antigen umfasst.

21. Verwendung eines HIV-lentiviralen Vektorpartikels nach einem beliebigen der Ansprüche 1 bis 20, wobei der lentivirale Vektor mit einem VSV-G-Protein pseudotypisiert ist.

22. Verwendung eines HIV-lentiviralen Vektorpartikels nach einem beliebigen der Ansprüche 1 bis 21, wobei das VSV-G-Protein die Transmembrandomäne des Indiana-VSV-G und die Ektodomäne des New-Jersey-Serotyps umfasst oder daraus besteht.

23. Verwendung eines HIV-lentiviralen Vektorpartikels nach einem beliebigen der Ansprüche 1 bis 22, wobei die Immunantwort eine frühe Immunantwort ist.

24. Verwendung eines HIV-lentiviralen Vektorpartikels nach Anspruch 23, wobei die Immunantwort so kurz wie eine Woche nach Verabreichung der Zusammensetzung induziert wird.

25. Verwendung eines HIV-lentiviralen Vektorpartikels nach einem beliebigen der Ansprüche 1 bis 24, wobei die Immunantwort eine langanhaltende Immunantwort ist.

26. Verwendung eines HIV-lentiviralen Vektorpartikels nach Anspruch 25, wobei die Immunantwort noch mindestens zwei Monate nach der Verabreichung der Zusammensetzung protektiv ist.

27. Verwendung eines HIV-lentiviralen Vektorpartikels nach einem beliebigen der Ansprüche 1 bis 26, wobei die Immunantwort im Wirt mit einer einzigen Verabreichung erreicht wird.

28. Verwendung eines HIV-lentiviralen Vektorpartikels nach einem beliebigen der Ansprüche 1 bis 27, die in Dosen des HIV-lentiviralen Vektors, die sich im Bereich von 1 bis 50 µg, stärker bevorzugt 1 bis 10 µg befinden, verabreicht wird.

29. Verwendung eines HIV-lentiviralen Vektorpartikels nach einem beliebigen der Ansprüche 1 bis 28, wobei die Zusammensetzung durch eine subkutane (s.c.), intradermale (i.d.), intramukuläre (i.m.) oder intravenöse (i.v.) Injektion, eine orale Verabreichung und eine mukosale Verabreichung, insbesondere intranasale Verabreichung oder Inhalation verabreicht wird.

30. Verwendung eines HIV-lentiviralen Vektorpartikels nach einem beliebigen der Ansprüche 1 bis 29, um Infektion des Wirts durch ein Pathogen wie ein Virus, ein Bakterium oder einen Parasit zu verhindern.

## Revendications

1. Utilisation d'une particule de vecteur lentiviral VIH non intégratif comprenant une protéine intégrase défectueuse et comprenant en outre un génome d'ARN qui est le produit de transcription d'un génome de vecteur comprenant un polynucléotide codant pour au moins un polypeptide antigénique, deux LTR et un ADN Flap comprenant un domaine cPPT et un domaine CTS, dans la fabrication d'une composition immunogène pour susciter une réponse immunitaire protectrice contre ledit ou lesdits polypeptides antigéniques pour prévenir une infection par un agent pathogène ou des états malins, chez un être humain auquel est administrée ladite composition immunogène comprenant des doses desdites particules de vecteur lentiviral VIH allant de 1 µg à 100 µg d'antigène p24, dans laquelle ladite protéine intégrase défectueuse est une intégrase ayant une mutation de classe I pour modifier spécifiquement son activité intégrase.

2. Utilisation d'une particule de vecteur lentiviral VIH selon la revendication 1, dans laquelle ledit vecteur lentivirus est non réplicatif.

3. Utilisation d'une particule de vecteur lentiviral VIH selon l'une quelconque des revendications 1 à 2, dans laquelle ladite réponse immunitaire est une réponse immunitaire humorale protectrice.

4. Utilisation d'une particule de vecteur lentiviral VIH selon l'une quelconque des revendications 1 à 2, dans laquelle ladite réponse immunitaire est une réponse immunitaire cellulaire.

5. Utilisation d'une particule de vecteur lentiviral VIH selon la revendication 4, dans laquelle ladite réponse immunitaire est une réponse immunitaire cellulaire médiée par CD8.

6. Utilisation d'une particule de vecteur lentiviral VIH selon la revendication 4, dans laquelle ladite réponse immunitaire est une réponse immunitaire cellulaire médiée par CD4.

7. Utilisation d'une particule de vecteur lentiviral VIH selon la revendication 1, dans laquelle ladite mutation est D64V.

8. Utilisation d'une particule de vecteur lentiviral VIH selon l'une quelconque des revendications 1 à 7, dans laquelle le génome lentiviral est dépourvu de gènes lentiviraux gag, pol et/ou env.

9. Utilisation d'une particule de vecteur lentiviral VIH selon l'une quelconque des revendications 1 à 7, dans laquelle le génome lentiviral est dépourvu de gènes fonctionnels lentiviraux gag, pol et/ou env.

10. Utilisation d'une particule de vecteur lentiviral VIH selon l'une quelconque des revendications 1 à 8, dans laquelle le génome lentiviral est dépourvu de toutes les séquences lentivirales codantes endogènes.

11. Utilisation d'une particule de vecteur lentiviral VIH selon l'une quelconque des revendications 1 à 10, dans laquelle le génome lentiviral comprend (a) une LTR5' et une LTR3', (b) une séquence psi, (c) un ADN Flap comprenant un domaine cPPT et un domaine CTS et (d) une séquence codant pour ledit ou lesdits polypeptides.

12. Utilisation d'une particule de vecteur lentiviral VIH selon la revendication 11, dans laquelle ledit génome lentiviral comprend en outre un site donneur d'épissage (SD), un site accepteur d'épissage (SA) et un élément sensible à Rev (RRE).

13. Utilisation d'une particule de vecteur lentiviral VIH selon la revendication 11 à 12, dans laquelle ledit génome lentiviral comprend en outre une origine de réplication (ori).

14. Utilisation d'une particule de vecteur lentiviral VIH selon l'une quelconque des revendications 11 à 13, dans laquelle ledit génome lentiviral comprend en outre une région de fixation d'échafaudage (SAR) et/ou une région de fixation de matrice (MAR).

15. Utilisation d'une particule de vecteur lentiviral VIH selon l'une quelconque des revendications 1 à 14, dans laquelle la LTR est délétée pour le promoteur et l'amplificateur de U3.

16. Utilisation d'une particule de vecteur lentiviral VIH selon l'une quelconque des revendications 1 à 15, dans laquelle l'ADN Flap comprend ou est constitué des séquences telles que définies dans SEQ ID NO: 1 à 7, comme représenté sur la figure 6.

17. Utilisation d'une particule de vecteur lentiviral VIH selon l'une quelconque des revendications 1 à 16, dans laquelle ledit au moins un polypeptide est codé par une séquence issue du génome d'un agent pathogène.

18. Utilisation d'une particule de vecteur lentiviral VIH selon la revendication 17, dans laquelle ledit agent pathogène est un virus, en particulier un rétrovirus, un lentivirus, un flavivirus ou un coronavirus, une bactérie ou un parasite.

19. Utilisation d'une particule de vecteur lentiviral VIH selon la revendication 18, dans laquelle ledit ou lesdits polypeptides sont dérivés de l'enveloppe de virus du sida, incluant le VIH-1 ou le VIH-2, de la capside du VIH ou de l'enveloppe du virus de la fièvre jaune, du virus du Nil occidental, du virus de la dengue (DV), du virus de l'encéphalite japonaise (JEV) ou du coronavirus associé au SRAS.

20. Utilisation d'une particule de vecteur lentiviral VIH selon l'une quelconque des revendications 1 à 16, dans laquelle ledit ou lesdits polypeptides comprennent un épitope ou un antigène tumoral.

21. Utilisation d'une particule de vecteur lentiviral VIH selon l'une quelconque des revendications 1 à 20, dans laquelle ledit vecteur lentiviral est pseudotypé avec une protéine VSV-G.

22. Utilisation d'une particule de vecteur lentiviral VIH selon l'une quelconque des revendications 1 à 21, dans laquelle ladite protéine VSV-G comprend ou est constituée du domaine transmembranaire de VSV-G d'Indiana et de l'ectodomaine du sérotype du New Jersey.

23. Utilisation d'une particule de vecteur lentiviral VIH selon l'une quelconque des revendications 1 à 22, dans laquelle ladite réponse immunitaire est une réponse immunitaire précoce.

24. Utilisation d'une particule de vecteur lentiviral VIH selon la revendication 23, dans laquelle ladite réponse immunitaire est induite en un temps aussi court qu'une semaine après l'administration de la composition.

25. Utilisation d'une particule de vecteur lentiviral VIH selon l'une quelconque des revendications 1 à 24, dans laquelle ladite réponse immunitaire est une réponse immunitaire durable.

26. Utilisation d'une particule de vecteur lentiviral VIH selon la revendication 25, dans laquelle ladite réponse immunitaire est encore protectrice au moins deux mois après l'administration de la composition.

27. Utilisation d'une particule de vecteur lentiviral VIH selon l'une quelconque des revendications 1 à 26, dans laquelle ladite réponse immunitaire chez ledit hôte est atteinte avec une seule administration.

28. Utilisation d'une particule de vecteur lentiviral VIH selon l'une quelconque des revendications 1 à 27, qui est administrée en doses dudit vecteur lentiviral VIH allant de 1 à 50 µg, plus préférablement de 1 à 10 µg.

29. Utilisation d'une particule de vecteur lentiviral VIH selon l'une quelconque des revendications 1 à 28, dans laquelle ladite composition est administrée par une injection sous-cutanée (s.c.), intradermique (i.d.), intramusculaire (i.m.) ou intraveineuse (i.v.), une administration orale et une administration muqueuse, en particulier une administration intranasale ou une inhalation.

30. Utilisation d'une particule de vecteur lentiviral VIH selon l'une quelconque des revendications 1 à 29, pour prévenir l'infection de l'hôte par un agent pathogène tel qu'un virus, une bactérie ou un parasite.
